# EUROPEAN PATENT SPECIFICATION

(11) **EP 2 303 917 B1**
(45) Date of publication and mention of the grant of the patent: **11.11.2020**
(21) Application number: 09770553.7
(22) Date of filing: 26.06.2009
(51) Int. Cl.: A61K 47/68, A61K 38/17, A61P 7/06

(54) **ANTAGONISTS OF ACTRIIB AND USES FOR INCREASING RED BLOOD CELL LEVELS**
ANTAGONISTEN VON ACTRIIB UND ANWENDUNGEN ZUR ERHÖHUNG DER KONZENTRATION AN ROTEN BLUTKÖRPERCHEN
ANTAGONISTES D'ACTRIIB ET UTILISATIONS POUR AUGMENTER LES TAUX
D'ÉRYTHROCYTES

(30) Priority: 26.06.2008 US 133368 P
(43) Date of publication of application: 06.04.2011
(60) Divisional application: 20195139.9
(73) Proprietor: Acceleron Pharma Inc., Cambridge, MA 02139 (US)
(72) Inventor: SHERMAN, Matthew, L., Newton, MA 02459 (US); SEEHRA, Jasbir, Lexington, MA 02421-6818 (US); BORGSTEIN, Niels, Medfield, MA 02052 (US)
(74) Representative: Lee, Nicholas John
(86) International application number: PCT/US2009/003808
(87) International publication number: WO 2009/158015

(56) References cited:
- WO-A1-92/04913
- WO-A2-2004/039948
- WO-A2-2008/076437
- US-A1- 2006 068 468
- US-A1- 2007 184 052
- US-A1- 2007 275 895
- US-B1- 6 537 966
- US-B2- 6 451 334
- LI QINGLEI ET AL: "Prevention of cachexia-like syndrome development and reduction of tumor progression in inhibin-deficient mice following administration of a chimeric activin receptor type II-murine Fc protein", MOLECULAR HUMAN REPRODUCTION, OXFORD UNIVERSITY PRESS, GB - BE, vol. 13, no. 9, 18 August 2007 (2007-08-18), pages 675-683, XP009102916, ISSN: 1360-9947, DOI: 10.1093/MOLEHR/GAM055
- MAGUER-SATTA V ET AL: "A novel role for fibronectin type I domain in the regulation of human hematopoietic cell adhesiveness through binding to follistatin domains of FLRG and follistatin", EXPERIMENTAL CELL RESEARCH, ACADEMIC PRESS, US, vol. 312, no. 4, 15 February 2006 (2006-02-15), pages 434-442, XP024944910, ISSN: 0014-4827, DOI: 10.1016/J.YEXCR.2005.11.006 [retrieved on 2006-02-15]
- MAGUER-SATTA V ET AL: "FLRG, member of the follistatin family, a new player in hematopoiesis", MOLECULAR AND CELLULAR ENDOCRINOLOGY, ELSEVIER IRELAND LTD, IE, vol. 225, no. 1-2, 15 October 2004 (2004-10-15), pages 109-118, XP004586244, ISSN: 0303-7207, DOI: 10.1016/J.MCE.2004.07.009

## Description

### BACKGROUND OF THE INVENTION

The mature red blood cell, or erythrocyte, is responsible for oxygen transport in the circulatory systems of vertebrates. Red blood cells carry high concentrations of hemoglobin, a protein that binds oxygen in the lungs at relatively high partial pressure of oxygen (pO₂) and delivers oxygen to areas of the body with a relatively low pO₂.

Mature red blood cells are produced from pluripotent hematopoietic stem cells in a process termed erythropoiesis. In post-natal individuals, erythropoiesis occurs primarily in the bone marrow and in the red pulp of the spleen. The coordinated action of various signaling pathways control the balance of cell proliferation, differentiation, survival and death. Under normal conditions, red blood cells are produced at a rate that maintains a constant red cell mass in the body, and production may increase or decrease in response to various stimuli, including increased or decreased oxygen tension or tissue demand. The process of erythropoiesis begins with the formation of lineage committed precursor cells and proceeds through a series of distinct precursor cell types. The final stages of erythropoiesis occur as reticulocytes are released into the bloodstream and lose their mitochondria and ribosomes while assuming the morphology of mature red blood cell. An elevated level of reticulocytes, or an elevated reticulocyte:erythrocyte ratio, in the blood is indicative of increased red blood cell production rates.

Erythropoietin (Epo) is widely recognized as the most significant positive regulator of erythropoiesis in post-natal vertebrates. Epo regulates the compensatory erythropoietic response to reduced tissue oxygen tension (hypoxia) and low red blood cell levels or low hemoglobin levels. In humans, elevated Epo levels promote red blood cell formation by stimulating the generation of erythroid progenitors in the bone marrow and spleen. In the mouse, Epo enhances erythropoiesis primarily in the spleen.

Various forms of recombinant Epo are used by physicians to increase red blood cell levels in a variety of clinical settings, and particularly for the treatment of anemia. Anemia is a broadly-defined condition characterized by lower than normal levels of hemoglobin or red blood cells in the blood. In some instances, anemia is caused by a primary disorder in the production or survival of red blood cells. More commonly, anemia is secondary to diseases of other systems (Weatherall & Provan (2000) Lancet 355, 1169-1175). Anemia may result from a reduced rate of production or increased rate of destruction of red blood cells or by loss of red blood cells due to bleeding. Anemia may result from a variety of disorders that include, for example, chronic renal failure, myelodysplastic syndrome, rheumatoid arthritis, and bone marrow transplantation.

Treatment with Epo typically causes a rise in hemoglobins by about 1-3 g/dL in healthy humans over a period of weeks. When administered to anemic individuals, this treatment regimen often provides substantial increases in hemoglobin and red blood cell levels and leads to improvements in quality of life and prolonged survival. Epo is not uniformly effective, and many individuals are refractory to even high doses (Horl et al. (2000) Nephrol Dial Transplant 15, 43-50). Over 50% of patients with cancer have an inadequate response to Epo, approximately 10% with end-stage renal disease are hyporesponsive (Glaspy et al. (1997) J Clin Oncol 15, 1218-1234; Demetri et al. (1998) J Clin Oncol 16, 3412-3425), and less than 10% with myelodysplastic syndrome respond favorably (Estey (2003) Curr Opin Hematol 10, 60-67). Several factors, including inflammation, iron and vitamin deficiency, inadequate dialysis, aluminum toxicity, and hyperparathyroidism may predict a poor therapeutic response, the molecular mechanisms of resistance to Epo are as yet unclear.

Thus, it is an object of the present disclosure to provide alternative compositions and methods for increasing red blood cell levels in patients.

### SUMMARY OF THE INVENTION

The scope for which protection is sought is as defined in the claims.

In part, the disclosure provides ActRIIb antagonists that can be used to increase red blood cell and hemoglobin levels. In particular, the disclosure demonstrates that a soluble form of ActRIIb, which acts as an inhibitor of activin or myostatin or other ActRIIb ligands, stimulates erythropoiesis activites (e.g., increases reticulocytes, increases mature and immature erythroid progenitors, etc.) when administered in vivo. While soluble ActRIIb may affect red blood cell levels through a mechanism other than activin or myostatin antagonism, the disclosure nonetheless demonstrates that desirable therapeutic agents may be selected on the basis of activin antagonism, myostatin antagonist or ActRIIb antagonism or any of the foregoing. As described in U.S. Publication No. 2009/0005308, ActRIIb antagonists can be used to promote muscle growth and increase muscle strength. Accordingly, the disclosure provides methods for promoting muscle growth and increasing red blood cell levels, particularly in patients with disorders that are characterized by anemia and loss of muscle, such as cancer- and cancer treatment-related muscle loss, many forms of cachexia and sarcopenia (muscle loss associated with aging). ActRIIb antagonists may also be used to promote bone growth and increase red blood cells in patients in need thereof, such as patients with osteoporosis and anemia, or patients with cancers (or recipients of chemotherapy treatments) associated with bone loss and anemia

In certain aspects, the disclosure provides polypeptides comprising a soluble, ligand-binding ActRIIb polypeptide that binds to activin or myostatin or other ActRIIb ligand. ActRIIb polypeptides may be formulated as a pharmaceutical preparation comprising the ligand-binding (e.g. activin-binding) ActRIIb polypeptide and a pharmaceutically acceptable carrier. The ligand-binding ActRIIb polypeptide may bind to activin with a K_{D} less than 1 micromolar or less than 100, 10 or 1 nanomolar. The composition may be at least 95% pure, with respect to other polypeptide components, as assessed by size exclusion chromatography, and optionally, the composition is at least 98% pure. An ActRIIb polypeptide for use in such a preparation may be any of those disclosed herein, such as a polypeptide having an amino acid sequence selected from SEQ ID NOs: 2, 3, 6, 8, or 9 or having an amino acid sequence that is at least 80%, 85%, 90%, 95%, 97% or 99% identical to an amino acid sequence selected from SEQ ID NOs: 2, 3, 6, 8, or 9. An active ActRIIb polypeptide may include a functional fragment of a natural ActRIIb polypeptide, such as one comprising at least 10, 20 or 30 amino acids of SEQ ID NOs: 1-3 or a sequence lacking the C-terminal 10 to 15 amino acids (the "tail") such as SEQ ID NO: 3.

A soluble, ligand-binding (e.g., activin-binding) ActRIIb polypeptide may include one or more alterations in the amino acid sequence (e.g., in the ligand-binding domain) relative to a naturally occurring ActRIIb polypeptide. Examples of altered ActRIIb polypeptides are provided in WO 2006/012627, pp. 59-60 and pp. 55-58, respectively, and throughout U.S. Patent Application Serial No. 12/012,652. The alteration in the amino acid sequence may, for example, alter glycosylation of the polypeptide when produced in a mammalian, insect or other eukaryotic cell or alter proteolytic cleavage of the polypeptide relative to the naturally occurring ActRIIb polypeptide.

A ligand-binding (e.g., activin-binding) ActRIIb polypeptide may be a fusion protein that has, as one domain, an ActRIIb polypeptide, (e.g., a ligand-binding portion of an ActRIIb) and one or more additional domains that provide a desirable property, such as improved pharmacokinetics, easier purification, targeting to particular tissues, etc. For example, a domain of a fusion protein may enhance one or more of in vivo stability, in vivo half life, uptake/administration, tissue localization or distribution, formation of protein complexes, multimerization of the fusion protein, and/or purification. A ligand-binding ActRIIb fusion protein may include an immunoglobulin Fc domain (wild-type or mutant) or a serum albumin or other polypeptide portion that provides desirable properties such as improved pharmacokinetics, improved solubility or improved stability. In a preferred embodiment, an ActRIIb -Fc fusion comprises a relatively unstructured linker positioned between the Fc domain and the extracellular ActRIIb domain. This unstructured linker may be an artificial sequence of 1, 2, 3, 4 or 5 amino acids or a length of between 5 and 15, 20, 30, 50 or more amino acids that are relatively free of secondary structure, or a mixture of both. A linker may be rich in glycine and proline residues and may, for example, contain a single sequence of threonine/serine and glycines or repeating sequences of threonine/serine and glycines (e.g., TG₄ (SEQ ID NO: 14) or SG₄ (SEQ ID NO: 15) singlets or repeats). A fusion protein may include a purification subsequence, such as an epitope tag, a FLAG tag, a polyhistidine sequence, and a GST fusion. Optionally, a soluble ActRIIb polypeptide includes one or more modified amino acid residues selected from: a glycosylated amino acid, a PEGylated amino acid, a farnesylated amino acid, an acetylated amino acid, a biotinylated amino acid, an amino acid conjugated to a lipid moiety, and an amino acid conjugated to an organic derivatizing agent. A pharmaceutical preparation may also include one or more additional compounds such as a compound that is used to treat a bone disorder, muscle disorder or a compound that is used to treat anemia. Preferably, a pharmaceutical preparation is substantially pyrogen free. In general, it is preferable that an ActRIIb protein be expressed in a mammalian cell line that mediates suitably natural glycosylation of the ActRIIb protein so as to diminish the likelihood of an unfavorable immune response in a patient. Human and CHO cell lines have been used successfully, and it is expected that other common mammalian expression systems will be useful.

In some embodiments, ActRIIb proteins designated ActRIIb-Fc have specific properties, including selective binding to activin versus GDF8 and/or GDF11 or vice versa, high affinity ligand binding and serum half life greater than two weeks in animal models and in human patients. In certain embodiments the invention provides ActRIIb-Fc polypeptides and pharmaceutical preparations comprising such polypeptides and a pharmaceutically acceptable excipient.

In certain aspects, the disclosure provides nucleic acids encoding a soluble ligand-binding ActRIIb polypeptide. An isolated polynucleotide may comprise a coding sequence for a soluble, ligand-binding (e.g. activin-binding) ActRIIb polypeptide, such as described above. For example, an isolated nucleic acid may include a sequence coding for an extracellular domain (e.g., ligand-binding domain) of an ActRIIb and a sequence that would code for part or all of the transmembrane domain and/or the cytoplasmic domain of an ActRIIb, but for a stop codon positioned within the transmembrane domain or the cytoplasmic domain, or positioned between the extracellular domain and the transmembrane domain or cytoplasmic domain. For example, an isolated polynucleotide may comprise a full-length ActRIIb polynucleotide sequence such as SEQ ID NO: 4 or a partially truncated version of ActRIIb, such as a nucleic acid comprising the nucleic acid sequence of SEQ ID NO: 5 which corresponds to the extracellular domain of ActRIIb. An isolated polynucleotide may further comprise a transcription termination codon at least six hundred nucleotides before the 3'-terminus or otherwise positioned such that translation of the polynucleotide gives rise to an extracellular domain optionally fused to a truncated portion of a full-length ActRIIb. A preferred nucleic acid sequence for ActRIIb is SEQ ID NO: 10. Nucleic acids disclosed herein may be operably linked to a promoter for expression, and the disclosure provides cells transformed with such recombinant polynucleotides. Preferably the cell is a mammalian cell such as a CHO cell.

In certain aspects, the disclosure provides methods for making a soluble, ligand-binding (e.g. activin-binding) ActRIIb polypeptide. Such a method may include expressing any of the nucleic acids (e.g., SEQ ID NOs: 4, 5, or 10) disclosed herein in a suitable cell, such as a Chinese hamster ovary (CHO) cellor a human cell. Such a method may comprise: a) culturing a cell under conditions suitable for expression of the soluble ActRIIb polypeptide, wherein said cell is transformed with a soluble ActRIIb expression construct; and b) recovering the soluble ActRIIb polypeptide so expressed. Soluble ActRIIb polypeptides may be recovered as crude, partially purified or highly purified fractions. Purification may be achieved by a series of purification steps, including, for example, one, two or three or more of the following, in any order: protein A chromatography, anion exchange chromatography (e.g., Q sepharose), hydrophobic interaction chromatography (e.g., phenylsepharose), size exclusion chromatography, and cation exchange chromatography. Soluble ActRIIb polypeptides may be formulated in liquid or solid (e.g., lyophilized) forms.

In certain aspects, an ActRIIb antagonist disclosed herein may be used in a method for promoting red blood cell production or increasing red blood cell levels in a subject. The disclosure provides methods for treating a disorder associated with low red blood cell counts or low hemoglobin levels (e.g., an anemia), or to promote red blood cell production, in patients in need thereof. A method may comprise administering to a subject in need thereof an effective amount of ActRIIb antagonist. The disclosure provides methods for increasing red blood cell levels and promoting muscle growth or increasing muscle strength in a patient in need thereof. The disclosure demonstrates that, in rodents, ActRIIb antagonists increase erythroid precursor cell levels primarily through effects on the spleen. Accordingly, the disclosure provides methods for increasing the release of red blood cells from the spleen, the method comprising administering to the patient an effective amount of an ActRIIb antagonist. In certain aspects, the disclosure provides uses of ActRIIb antagonists for making a medicament for the treatment of a disorder or condition as described herein.

In certain aspects, the disclosure provides a method for identifying an agent that stimulates production of red blood cells. The method comprises: a) identifying a test agent that binds to activin or a ligand-binding domain of an ActRIIb polypeptide; and b) evaluating the effect of the agent on the levels of red blood cells, hemoglobin, and/or red blood cell precursor levels (e.g., reticulocyte levels).

### BRIEF DESCRIPTION OF THE DRAWINGS

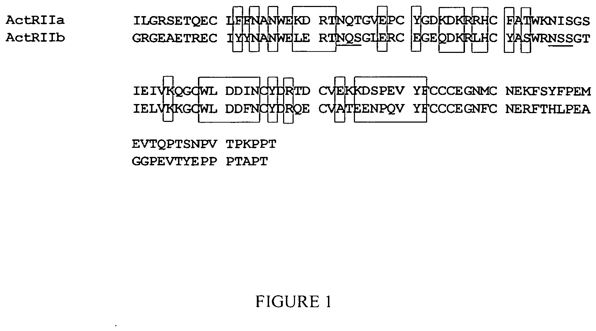

Figure 1 shows an alignment of human ActRIIA and ActRIIB with the residues that are deduced herein, based on composite analysis of multiple ActRIIB and ActRIIA crystal structures to directly contact ligand (the ligand binding pocket) indicated with boxes.

### DETAILED DESCRIPTION OF THE INVENTION

### 1. Overview

The transforming growth factor-beta (TGF-beta) superfamily contains a variety of growth factors that share common sequence elements and structural motifs. These proteins are known to exert biological effects on a large variety of cell types in both vertebrates and invertebrates. Members of the superfamily perform important functions during embryonic development in pattern formation and tissue specification and can influence a variety of differentiation processes, including adipogenesis, myogenesis, chondrogenesis, cardiogenesis, hematopoiesis, neurogenesis, and epithelial cell differentiation. The family is divided into two general branches: the BMP/GDF and the TGF-beta/Activin/BMP10 branches, whose members have diverse, often complementary effects. By manipulating the activity of a member of the TGF-beta family, it is often possible to cause significant physiological changes in an organism. For example, the Piedmontese and Belgian Blue cattle breeds carry a loss-of-function mutation in the GDF8 (also called myostatin) gene that causes a marked increase in muscle mass. Grobet et al., Nat Genet. 1997, 17(1):71-4. Furthermore, in humans, inactive alleles of GDF8 are associated with increased muscle mass and, reportedly, exceptional strength. Schuelke et al., N Engl J Med 2004, 350:2682-8.

Activins are dimeric polypeptide growth factors that belong to the TGF-beta superfamily. There are three principal activin forms (A, B, and AB) that are homo/heterodimers of two closely related β subunits (β_{A}β_{A}, β_{B}β_{B}, and β_{A}β_{B}, respectively). The human genome also encodes an activin C and an activin E, which are primarily expressed in the liver, and heterodimeric forms containing β_{C} or β_{E} are also known. In the TGF-beta superfamily, activins are unique and multifunctional factors that can stimulate hormone production in ovarian and placental cells, support neuronal cell survival, influence cell-cycle progress positively or negatively depending on cell type, and induce mesodermal differentiation at least in amphibian embryos (DePaolo et al., 1991, Proc Soc Ep Biol Med. 198:500-512; Dyson et al., 1997, Curr Biol. 7:81-84; Woodruff, 1998, Biochem Pharmacol. 55:953-963). Moreover, erythroid differentiation factor (EDF) isolated from the stimulated human monocytic leukemic cells was found to be identical to activin A (Murata et al., 1988, PNAS, 85:2434). It has been suggested that activin A promotes erythropoiesis in the bone marrow. In several tissues, activin signaling is antagonized by its related heterodimer, inhibin. For example, during the release of follicle-stimulating hormone (FSH) from the pituitary, activin promotes FSH secretion and synthesis, while inhibin prevents FSH secretion and synthesis. Other proteins that may regulate activin bioactivity and/or bind to activin include follistatin (FS), follistatin-related protein (FSRP) and α₂-macroglobulin.

TGF-β signals are mediated by heteromeric complexes of type I and type II serine/ threonine kinase receptors, which phosphorylate and activate downstream Smad proteins upon ligand stimulation (Massagué, 2000, Nat. Rev. Mol. Cell Biol. 1:169-178). These type I and type II receptors are transmembrane proteins, composed of a ligand-binding extracellular domain with cysteine-rich region, a transmembrane domain, and a cytoplasmic domain with predicted serine/threonine specificity. Type I receptors are essential for signaling; and type II receptors are required for binding ligands and for expression of type I receptors. Type I and II activin receptors form a stable complex after ligand binding, resulting in phosphorylation of type I receptors by type II receptors.

Two related type II receptors (ActRII), ActRIIa and ActRIIb, have been identified as the type II receptors for activins (Mathews and Vale, 1991, Cell 65:973-982; Attisano et al., 1992, Cell 68: 97-108). Besides activins, ActRIIa and ActRIIb can biochemically interact with several other TGF-β family proteins, including BMP7, Nodal, GDF8, and GDF11 (Yamashita et al., 1995, J. Cell Biol. 130:217-226; Lee and McPherron, 2001, Proc. Natl. Acad. Sci. 98:9306-9311; Yeo and Whitman, 2001, Mol. Cell 7: 949-957; Oh et al., 2002, Genes Dev. 16:2749-54). ALK4 is the primary type I receptor for activins, particularly for activin A, and ALK-7 may serve as a receptor for activins as well, particularly for activin B.

As demonstrated herein, a soluble ActRIIb polypeptide (sActRIIb) is effective to increase reticulocyte levels in vivo, an effect which, over a longer time period is expected to cause increased hematocrit levels. Thus, sActRIIb polypeptides of the disclosure may be used to increase red blood cell levels in vivo. As shown herein, ActRIIb antagonists stimulate erythropoiesis in rodents and monkeys. It should be noted that hematopoiesis is a complex process, regulated by a variety of factors, including erythropoietin, G-CSF and iron homeostasis. The terms "increase red blood cell levels" and "promote red blood cell formation" refer to clinically observable metrics, such as hematocrit, red blood cell counts and hemoglobin measurements, and are intended to be neutral as to the mechanism by which such changes occur.

In addition to stimulating red blood cell levels, certain ActRIIb antagonists are useful for a variety of therapeutic applications, including, for example, promoting bone growth (see PCT Publication WO 2006/012627) and promoting muscle growth (see PCT Publication No. WO2006/ 012627 and PCT Application No. PCT/US2008/001506). ActRIIb antagonists include, for example, ligand-binding (e.g. activin-binding) soluble ActRIIb polypeptides, , antibodies that bind to ActRIIb and disrupt activin binding, non-antibody proteins selected for ActRIIb binding (see e.g., WO/2002/088171, WO/2006/055689, and WO/2002/032925 for examples of such proteins and methods for design and selection of same), randomized peptides selected for ActRIIb binding, often affixed to an Fc domain. Two different proteins (or other moieties) with ActRIIb binding activity may be linked together to create a bifunctional binding molecule. Nucleic acid aptamers, small molecules and other agents that inhibit the ActRIIb signaling axis are included as ActRIIb antagonists. Various proteins have antagonist that may be similar to ActRIIb antagonists, including inhibin (i.e., inhibin alpha subunit), although inhibin does not universally antagonize activin in all tissues, follistatin (e.g., follistatin-288 and follistatin-315), FSRP, FLRG, activin C, alpha(2)-macroglobulin, and an M108A (methionine to alanine change at position 108) mutant activin A. Generally, alternative forms of activin, particularly those with alterations in the type I receptor binding domain can bind to type II receptors and fail to form an active ternary complex, thus acting as antagonists. Additionally, nucleic acids, such as antisense molecules, siRNAs or ribozymes that inhibit ActRIIb expression, can be used as ActRIIb antagonists. The ActRIIb antagonist to be used may exhibit selectivity for inhibiting activin-mediated signaling versus other members of the TGF-beta family, and particularly with respect to GDF8 and GDF11.

The terms used in this specification generally have their ordinary meanings in the art, within the context of this invention and in the specific context where each term is used. Certain terms are discussed below or elsewhere in the specification, to provide additional guidance to the practitioner in describing the compositions of the invention and methods of the disclosure and how to make and use them. The scope or meaning of any use of a term will be apparent from the specific context in which the term is used.

"About" and "approximately" shall generally mean an acceptable degree of error for the quantity measured given the nature or precision of the measurements. Typically, exemplary degrees of error are within 20 percent (%), preferably within 10%, and more preferably within 5% of a given value or range of values.

Alternatively, and particularly in biological systems, the terms "about" and "approximately" may mean values that are within an order of magnitude, preferably within 5-fold and more preferably within 2-fold of a given value. Numerical quantities given herein are approximate unless stated otherwise, meaning that the term "about" or "approximately" can be inferred when not expressly stated.

The methods of the disclosure may include steps of comparing sequences to each other, including wild-type sequence to one or more mutants (sequence variants). Such comparisons typically comprise alignments of polymer sequences, e.g., using sequence alignment programs and/or algorithms that are well known in the art (for example, BLAST, FASTA and MEGALIGN, to name a few). The skilled artisan can readily appreciate that, in such alignments, where a mutation contains a residue insertion or deletion, the sequence alignment will introduce a "gap" (typically represented by a dash, or "A") in the polymer sequence not containing the inserted or deleted residue.

"Homologous," in all its grammatical forms and spelling variations, refers to the relationship between two proteins that possess a "common evolutionary origin," including proteins from superfamilies in the same species of organism, as well as homologous proteins from different species of organism. Such proteins (and their encoding nucleic acids) have sequence homology, as reflected by their sequence similarity, whether in terms of percent identity or by the presence of specific residues or motifs and conserved positions.

The term "sequence similarity," in all its grammatical forms, refers to the degree of identity or correspondence between nucleic acid or amino acid sequences that may or may not share a common evolutionary origin.

However, in common usage and in the instant application, the term "homologous," when modified with an adverb such as "highly," may refer to sequence similarity and may or may not relate to a common evolutionary origin.

### 2. ActRIIb Polypeptides

In certain aspects, the present invention relates to ActRIIb polypeptides. As used herein, the term "ActRIIb" refers to a family of activin receptor type IIb (ActRIIb) proteins from any species and variants derived from such ActRIIb proteins by mutagenesis or other modification. Reference to ActRIIb herein is understood to be a reference to any one of the currently identified forms. Members of the ActRIIb family are generally transmembrane proteins, composed of a ligand-binding extracellular domain with a cysteine-rich region, a transmembrane domain, and a cytoplasmic domain with predicted serine/threonine kinase activity.

The term "ActRIIb polypeptide" includes polypeptides comprising any naturally occurring polypeptide of an ActRIIb family member as well as any variants thereof (including mutants, fragments, fusions, and peptidomimetic forms) that retain a useful activity. See, for example, WO/2006/012627. For example, ActRIIb polypeptides include polypeptides derived from the sequence of any known ActRIIb having a sequence at least about 80% identical to the sequence of an ActRIIb polypeptide, and optionally at least 85%, 90%, 95%, 97%, 99% or greater identity. For example, an ActRIIb polypeptide of the invention may bind to and inhibit the function of an ActRIIb protein and/or a ligand such as activin or myostatin. An ActRIIb polypeptide may be selected for activity in promoting red blood cell formation in vivo. Examples of ActRIIb polypeptides include human ActRIIb precursor polypeptide (SEQ ID NO: 1) and soluble human ActRIIb polypeptides (e.g., SEQ ID NO: 2, 3, 8 and 9).

The human ActRIIb precursor protein sequence is as follows:

MTAPWVALALLWGSLWPGSGRGEAETRECIYYNANWELERTNQ
SGLERCEGEQDKRLHCYASWANSSGTIELVKKGCWLDDFNCYD
RQECVATEENPQVYFCCCEGNFCNERFTHLPEAGGPEVTYEPP
PTAPTLLTVLAYSLLPIGGLSLIVLLAFWMYRHRKPPYGHVDI
HEDPGPPPPSPLVGLKPLQLLEIKARGRFGCVWKAQLMNDFVA
VKIFPLQDKQSWQSEREIFSTPGMKHENLLQFIAAEKRGSNLE
VELWLITAFHDKGSLTDYLKGNIITWNELCHVAETMSRGLSYL
HEDVPWCRGEGHKPSIAHRDFKSKNVLLKSDLTAVLADFGLAV
RFEPGKPPGDTHGQVGTRRYMAPEVLEGAINFQRDAFLRIDMY
AMGLVLWELVSRCKAADGPVDEYMLPFEEEIGQHPSLEELQEV

VVHKKMRPTIKDHWLKHPGLAQLCVTIEECWDHDAEARLSAGC
VEERVSLIRRSVNGTTSDCLVSLVTSVTNVDLPPKESSI (SEQ
ID NO: 1)

The signal peptide is single underlined; the extracellular domain is in bold and the potential N-linked glycosylation sites are in boxes.

The human ActRIIb soluble (extracellular), processed polypeptide sequence is as follows:

GRGEAETRECIYYNANWELERTNQSGLERCEGEQDKRLHCYAS
WANSSGTIELVKKGCWLDDFNCYDRQECVATEENPQVYFCCCE
GNFCNERFTHLPEAGGPEVTYEPPPTAPT (SEQ ID NO: 2)

In some conditions, the protein may be produced with an "SGR..." sequence at the N-terminus. The C-terminal "tail" of the extracellular domain is underlined. The sequence with the "tail" deleted (a Δ15 sequence) is as follows:

GRGEAETRECIYYNANWELERTNQSGLERCEGEQDKRLHCYAS
WANSSGTIELVKKGCWLDDFNCYDRQECVATEENPQVYFCCCE
GNFCNERFTHLPEA (SEQ ID NO: 3)

In some conditions, the protein may be produced with an "SGR..." sequence at the N-terminus. The nucleic acid sequence encoding a human ActRIIb precursor protein is as follows: (nucleotides 5-1543 of Genbank entry NM_001106)

ATGACGGCGCCCTGGGTGGCCCTCGCCCTCCTCTGGGGATCGC
TGTGGCCCGGCTCTGGGCGTGGGGAGGCTGAGACACGGAGTG
CATCTACTACAACGCCAACTGGGAGCTGGAGCGCACCAACCAG
AGCGGCCTGGAGCGCTGCGAAGGCGAGCAGGACAAGCGGCTGC
ACTGCTACGCCTCCTGGCCAACAGCTCTGGCACCATCGAGCT
CGTGAAGAAGGGCTGCTGGCTAGATGACTTCAACTGCTACGAT
AGGCAGGAGTGTGTGGCCACTGAGGAGAACCCCCAGGTGTACT
TCTGCTGCTGTGAAGGCAACTTCTGCAACGAGCGCTTCACTCA
TTTGCCAGAGGCTGGGGGCCCGGAAGTCACGTACGAGCCACCC
CCGACAGCCCCCACCCTGCTCACGGTGCTGGCCTACTCACTGC
TGCCCATCGGGGGCCTTTCCCTCATCGTCCTGCTGGCCTTTTG
GATGTACCGGCATCGCAAGCCCCCCTACGGTCATGTGGACATC
CATGAGGACCCTGGGCCTCCACCACCATCCCCTCTGGTGGGCC

TGAAGCCACTGCAGCTGCTGGAGATCAAGGCTCGGGGGCGCTT
TGGCTGTGTCTGGAAGGCCCAGCTCATGAATGACTTTGTAGCT
GTCAAGATCTTCCCACTCCAGGACAAGCAGTCGTGGCAGAGTG
AACGGGAGATCTTCAGCACACCTGGCATGAAGCACGAGAACCT
GCTACAGTTCATTGCTGCCGAGAAGCGAGGCTCCAACCTCGAA
GTAGAGCTGTGGCTCATCACGGCCTTCCATGACAAGGGCTCCC
TCACGGATTACCTCAAGGGGAACATCATCACATGGAACGAACT
GTGTCATGTAGCAGAGACGATGTCACGAGGCCTCTCATACCTG
CATGAGGATGTGCCCTGGTGCCGTGGCGAGGGCCACAAGCCGT
CTATTGCCCACAGGGACTTTAAAAGTAAGAATGTATTGCTGAA
GAGCGACCTCACAGCCGTGCTGGCTGACTTTGGCTTGGCTGTT
CGATTTGAGCCAGGGAAACCTCCAGGGGACACCCACGGACAGG
TAGGCACGAGACGGTACATGGCTCCTGAGGTGCTCGAGGGAGC
CATCAACTTCCAGAGAGATGCCTTCCTGCGCATTGACATGTAT
GCCATGGGGTTGGTGCTGTGGGAGCTTGTGTCTCGCTGCAAGG
CTGCAGACGGACCCGTGGATGAGTACATGCTGCCCTTTGAGGA
AGAGATTGGCCAGCACCCTTCGTTGGAGGAGCTGCAGGAGGTG
GTGGTGCACAAGAAGATGAGGCCCACCATTAAAGATCACTGGT
TGAAACACCCGGGCCTGGCCCAGCTTTGTGTGACCATCGAGGA
GTGCTGGGACCATGATGCAGAGGCTCGCTTGTCCGCGGGCTGT
GTGGAGGAGCGGGTGTCCCTGATTCGGAGGTCGGTCAACGGCA
CTACCTCGGACTGTCTCGTTTCCCTGGTGACCTCTGTCACCAA
TGTGGACCTGCCCCCTAAAGAGTCAAGCATCTAA (SEQ ID
NO: 4)

The nucleic acid sequence encoding a human ActRIIb soluble (extracellular) polypeptide is as follows:

TCTGGGCGTGGGGAGGCTGAGACACGGAGTGCATCTACTACA
ACGCCAACTGGGAGCTGGAGCGCACCAACCAGAGCGGCCTGGA
GCGCTGCGAAGGCGAGCAGGACAAGCGGCTGCACTGCTACGCC
TCCTGGGCCAACAGCTCTGGCACCATCGAGCTCGTGAAGAAGG
GCTGCTGGCTAGATGACTTCAACTGCTACGATAGGCAGGAGTG
TGTGGCCACTGAGGAGAACCCCCAGGTGTACTTCTGCTGCTGT
GAAGGCAACTTCTGCAACGAGCGCTTCACTCATTTGCCAGAGG

CTGGGGGCCCGGAAGTCACGTACGAGCCACCCCCGACAGCCCC
CACC (SEQ ID NO: 5)

In a specific embodiment, the invention relates to soluble ActRIIb polypeptides. As described herein, the term "soluble ActRIIb polypeptide" generally refers to polypeptides comprising an extracellular domain of an ActRIIb protein. The term "soluble ActRIIb polypeptide," as used herein, includes any naturally occurring extracellular domain of an ActRIIb protein as well as any variants thereof (including mutants, fragments and peptidomimetic forms). A ligand-binding (e.g. activin-binding) ActRIIb polypeptide is one that retains the ability to bind to activin, including, for example, activin AA, AB, BB, or forms that include a C or E subunit. Optionally, a ligand-binding (e.g. activin-binding) ActRIIb polypeptide will bind to activin AA with a dissociation constant of 1 nM or less. The extracellular domain of an ActRIIb protein binds to activin and other ligands, such as myostatin, and is generally soluble in physiological conditions, and thus can be termed a soluble, ligand-binding (e.g. activin-binding) ActRIIb polypeptide. Examples of soluble, ligand-binding (e.g. activin-binding) ActRIIb polypeptides include the soluble polypeptides illustrated in SEQ ID NOs: 2, 3, 8, and 9. SEQ ID NO: 8 is referred to as ActRIIb-hFc, and is described further in the Examples. Other examples of soluble, ligand-binding (e.g. activin-binding) ActRIIb polypeptides comprise a signal sequence in addition to the extracellular domain of an ActRIIb protein, for example, the honey bee mellitin leader sequence (SEQ ID NO: 11), the tissue plaminogen activator (TPA) leader (SEQ ID NO: 12) or the native ActRIIb leader (SEQ ID NO: 13). The ActRIIb-hFc polypeptide illustrated in SEQ ID NO: 9 uses a TPA leader.

Extensive analysis of structure function analysis of ActRIIb is provided in U.S. Pat. Appl. 12/012,652. Figure 1 shows amino acids that are involved in the ligand binding domain. ActRIIb residues likely to be in contact with ligands in the binding pocket have been defined. At these positions, it is expected that conservative mutations will be tolerated, although a K74A mutation is well-tolerated, as are R40A, K55A, F82A and mutations at position L79. R40 is a K in Xenopus, indicating that basic amino acids at this position will be tolerated. Q53 is R in bovine ActRIIB and K in Xenopus ActRIIB, and therefore amino acids including R, K, Q, N and H will be tolerated at this position. Outside of these residues, it is expected that modifications will be relatively well-tolerated, provided that such alterations do not disrupt the structure of the protein as a whole. It is readily apparent when a protein structure is disrupted because the protein will tend to express poorly or be degraded in the culture media. Thus, a general formula for an active ActRIIb variant protein is one that comprises amino acids 12-82 of SEQ ID NO: 2 respectively, but optionally beginning at a position ranging from 1-5 or 3-5 and ending at a position ranging from 110-116 or 110-115, respectively, and comprising no more than 1, 2, 5, 10 or 15 conservative amino acid changes in the ligand binding pocket, and zero, one or more non-conservative alterations at positions 40, 53, 55, 74, 79 and/or 82 in the ligand binding pocket. Such a protein may comprise an amino acid sequence that retains greater than 80%, 90%, 95% or 99% sequence identity to the sequence of amino acids 29-109 of SEQ ID NO: 2.

Functionally active fragments of ActRIIb polypeptides can be obtained by screening polypeptides recombinantly produced from the corresponding fragment of the nucleic acid encoding an ActRIIb polypeptide. In addition, fragments can be chemically synthesized using techniques known in the art such as conventional Merrifield solid phase f-Moc or t-Boc chemistry. The fragments can be produced (recombinantly or by chemical synthesis) and tested to identify those peptidyl fragments that can function as antagonists (inhibitors) of ActRIIb protein or signaling mediated by activin.

Functionally active variants of ActRIIb polypeptides can be obtained by screening libraries of modified polypeptides recombinantly produced from the corresponding mutagenized nucleic acids encoding an ActRIIb polypeptide. The variants can be produced and tested to identify those that can function as antagonists (inhibitors) of ActRIIb protein or signaling mediated by activin. In certain cases, a functional variant of the ActRIIb polypeptides comprises an amino acid sequence that is at least 75% identical to an amino acid sequence selected from SEQ ID NOs: 2 or 3. In certain cases, the functional variant has an amino acid sequence at least 80%, 85%, 90%, 95%, 97%, 98%, 99% or 100% identical to an amino acid sequence selected from SEQ ID NOs: 2 or 3.

Functional variants may be generated by modifying the structure of an ActRIIb polypeptide for such purposes as enhancing therapeutic efficacy, or stability (e.g., ex vivo shelf life and resistance to proteolytic degradation in vivo). Such modified ActRIIb polypeptides when selected to retain activin binding, are considered functional equivalents of the naturally-occurring ActRIIb polypeptides. Modified ActRIIb polypeptides can also be produced, for instance, by amino acid substitution, deletion, or addition. For instance, it is reasonable to expect that an isolated replacement of a leucine with an isoleucine or valine, an aspartate with a glutamate, a threonine with a serine, or a similar replacement of an amino acid with a structurally related amino acid (e.g., conservative mutations) will not have a major effect on the biological activity of the resulting molecule. Conservative replacements are those that take place within a family of amino acids that are related in their side chains. Whether a change in the amino acid sequence of an ActRIIb polypeptide results in a functional homolog can be readily determined by assessing the ability of the variant ActRIIb polypeptide to produce a response in cells in a fashion similar to the wild-type ActRIIb polypeptide.

In certain embodiments, the present invention contemplates specific mutations of the ActRIIb polypeptides so as to alter the glycosylation of the polypeptide. Such mutations may be selected so as to introduce or eliminate one or more glycosylation sites, such as O-linked or N-linked glycosylation sites. Asparagine-linked glycosylation recognition sites generally comprise a tripeptide sequence, asparagine-X-threonine or asparagine-X-serine (where "X" is any amino acid) which is specifically recognized by appropriate cellular glycosylation enzymes. The alteration may also be made by the addition of, or substitution by, one or more serine or threonine residues to the sequence of the wild-type ActRIIb polypeptide (for O-linked glycosylation sites). A variety of amino acid substitutions or deletions at one or both of the first or third amino acid positions of a glycosylation recognition site (and/or amino acid deletion at the second position) results in non-glycosylation at the modified tripeptide sequence. Another means of increasing the number of carbohydrate moieties on an ActRIIb polypeptide is by chemical or enzymatic coupling of glycosides to the ActRIIb polypeptide. Depending on the coupling mode used, the sugar(s) may be attached to (a) arginine and histidine; (b) free carboxyl groups; (c) free sulfhydryl groups such as those of cysteine; (d) free hydroxyl groups such as those of serine, threonine, or hydroxyproline; (e) aromatic residues such as those of phenylalanine, tyrosine, or tryptophan; or (f) the amide group of glutamine. Removal of one or more carbohydrate moieties present on an ActRIIb polypeptide may be accomplished chemically and/or enzymatically. Chemical deglycosylation may involve, for example, exposure of the ActRIIb polypeptide to the compound trifluoromethanesulfonic acid, or an equivalent compound. This treatment results in the cleavage of most or all sugars except the linking sugar (N-acetylglucosamine or N-acetylgalactosamine), while leaving the amino acid sequence intact. Enzymatic cleavage of carbohydrate moieties on ActRIIb polypeptides can be achieved by the use of a variety of endo- and exo-glycosidases as described by Thotakura et al. (1987) Meth. Enzymol. 138:350. The sequence of an ActRIIb polypeptide may be adjusted, as appropriate, depending on the type of expression system used, as mammalian, yeast, insect and plant cells may all introduce differing glycosylation patterns that can be affected by the amino acid sequence of the peptide. In general, ActRIIb proteins for use in humans may be expressed in a mammalian cell line that provides proper glycosylation, such as HEK293 or CHO cell lines, although other mammalian expression cell lines are expected to be useful as well. Other non-mammalian cell lines may be used (e.g., yeast, E. coli, insect cells), and in some cases, such cell lines may be engineered to include enzymes that confer mammalian-type glycosylation patterns on the expressed proteins.

This disclosure further contemplates a method of generating mutants, particularly sets of combinatorial mutants of an ActRIIb polypeptide, as well as truncation mutants; pools of combinatorial mutants are especially useful for identifying functional variant sequences. The purpose of screening such combinatorial libraries may be to generate, for example, ActRIIb polypeptide variants which bind to activin or other ligands. A variety of screening assays are provided below, and such assays may be used to evaluate variants. For example, an ActRIIb polypeptide variant may be screened for ability to bind to an ActRIIb ligand, to prevent binding of an ActRIIb ligand to an ActRIIb polypeptide or to interfere with signaling caused by an ActRIIb ligand.

The activity of an ActRIIb polypeptide or its variants may also be tested in a cell-based or in vivo assay. For example, the effect of an ActRIIb polypeptide variant on the expression of genes involved in hematopoiesis may be assessed. This may, as needed, be performed in the presence of one or more recombinant ActRIIb ligand proteins (e.g., activin), and cells may be transfected so as to produce an ActRIIb polypeptide and/or variants thereof, and optionally, an ActRIIb ligand. Likewise, an ActRIIb polypeptide may be administered to a mouse or other animal, and one or more blood measurements, such as an RBC count, hemoglobin, or reticulocyte count may be assessed.

Combinatorially-derived variants can be generated which have a selective or generally increased potency relative to a naturally occurring ActRIIb polypeptide. Likewise, mutagenesis can give rise to variants which have intracellular half-lives dramatically different than the corresponding a wild-type ActRIIb polypeptide. For example, the altered protein can be rendered either more stable or less stable to proteolytic degradation or other cellular processes which result in destruction of, or otherwise inactivation of a native ActRIIb polypeptide. Such variants, and the genes which encode them, can be utilized to alter ActRIIb polypeptide levels by modulating the half-life of the ActRIIb polypeptides. For instance, a short half-life can give rise to more transient biological effects and, when part of an inducible expression system, can allow tighter control of recombinant ActRIIb polypeptide levels within the cell. In an Fc fusion protein, mutations may be made in the linker (if any) and/or the Fc portion to alter the half-life of the protein.

A combinatorial library may be produced by way of a degenerate library of genes encoding a library of polypeptides which each include at least a portion of potential ActRIIb polypeptide sequences. For instance, a mixture of synthetic oligonucleotides can be enzymatically ligated into gene sequences such that the degenerate set of potential ActRIIb polypeptide nucleotide sequences are expressible as individual polypeptides, or alternatively, as a set of larger fusion proteins (e.g., for phage display).

There are many ways by which the library of potential homologs can be generated from a degenerate oligonucleotide sequence. Chemical synthesis of a degenerate gene sequence can be carried out in an automatic DNA synthesizer, and the synthetic genes can then be ligated into an appropriate vector for expression. The synthesis of degenerate oligonucleotides is well known in the art (see for example, Narang, SA (1983) Tetrahedron 39:3; Itakura et al., (1981) Recombinant DNA, Proc. 3rd Cleveland Sympos. Macromolecules, ed. AG Walton, Amsterdam: Elsevier pp273-289; Itakura et al., (1984) Annu. Rev. Biochem. 53:323; Itakura et al., (1984) Science 198:1056; Ike et al., (1983) Nucleic Acid Res. 11:477). Such techniques have been employed in the directed evolution of other proteins (see, for example, Scott et al., (1990) Science 249:386-390; Roberts et al., (1992) PNAS USA 89:2429-2433; Devlin et al., (1990) Science 249: 404-406; Cwirla et al., (1990) PNAS USA 87: 6378-6382; as well as U.S. Patent Nos: 5,223,409, 5,198,346, and 5,096,815).

Alternatively, other forms of mutagenesis can be utilized to generate a combinatorial library. For example, ActRIIb polypeptide variants can be generated and isolated from a library by screening using, for example, alanine scanning mutagenesis and the like (Ruf et al., (1994) Biochemistry 33:1565-1572; Wang et al., (1994) J. Biol. Chem. 269:3095-3099; Balint et al., (1993) Gene 137:109-118; Grodberg et al., (1993) Eur. J. Biochem. 218:597-601; Nagashima et al., (1993) J. Biol. Chem. 268:2888-2892; Lowman et al., (1991) Biochemistry 30:10832-10838; and Cunningham et al., (1989) Science 244:1081-1085), by linker scanning mutagenesis (Gustin et al., (1993) Virology 193:653-660; Brown et al., (1992) Mol. Cell Biol. 12:2644-2652; McKnight et al., (1982) Science 232:316); by saturation mutagenesis (Meyers et al., (1986) Science 232:613); by PCR mutagenesis (Leung et al., (1989) Method Cell Mol Biol 1:11-19); or by random mutagenesis, including chemical mutagenesis, etc. (Miller et al., (1992) A Short Course in Bacterial Genetics, CSHL Press, Cold Spring Harbor, NY; and Greener et al., (1994) Strategies in Mol Biol 7:32-34). Linker scanning mutagenesis, particularly in a combinatorial setting, is an attractive method for identifying truncated (bioactive) forms of ActRIIb polypeptides.

A wide range of techniques are known in the art for screening gene products of combinatorial libraries made by point mutations and truncations, and, for that matter, for screening cDNA libraries for gene products having a certain property. Such techniques will be generally adaptable for rapid screening of the gene libraries generated by the combinatorial mutagenesis of ActRIIb polypeptides. The most widely used techniques for screening large gene libraries typically comprises cloning the gene library into replicable expression vectors, transforming appropriate cells with the resulting library of vectors, and expressing the combinatorial genes under conditions in which detection of a desired activity facilitates relatively easy isolation of the vector encoding the gene whose product was detected. Preferred assays include activin binding assays and activin-mediated cell signaling assays.

In certain embodiments, the ActRIIb polypeptides of the invention may further comprise post-translational modifications in addition to any that are naturally present in the ActRIIb polypeptides. Such modifications include, but are not limited to, acetylation, carboxylation, glycosylation, phosphorylation, lipidation, and acylation. As a result, the modified ActRIIb polypeptides may contain non-amino acid elements, such as polyethylene glycols, lipids, poly- or mono-saccharide, and phosphates. Effects of such non-amino acid elements on the functionality of an ActRIIb polypeptide may be tested as described herein for other ActRIIb polypeptide variants. When an ActRIIb polypeptide is produced in cells by cleaving a nascent form of the ActRIIb polypeptide, post-translational processing may also be important for correct folding and/or function of the protein. Different cells (such as CHO, HeLa, MDCK, 293, WI38, NIH-3T3 or HEK293) have specific cellular machinery and characteristic mechanisms for such post-translational activities and may be chosen to ensure the correct modification and processing of the ActRIIb polypeptides.

In certain aspects, functional variants or modified forms of the ActRIIb polypeptides include fusion proteins having at least a portion of the ActRIIb polypeptides and one or more fusion domains. Well known examples of such fusion domains include, but are not limited to, polyhistidine, Glu-Glu, glutathione S transferase (GST), thioredoxin, protein A, protein G, an immunoglobulin heavy chain constant region (Fc), maltose binding protein (MBP), or human serum albumin. A fusion domain may be selected so as to confer a desired property. For example, some fusion domains are particularly useful for isolation of the fusion proteins by affinity chromatography. For the purpose of affinity purification, relevant matrices for affinity chromatography, such as glutathione-, amylase-, and nickel- or cobalt- conjugated resins are used. Many of such matrices are available in "kit" form, such as the Pharmacia GST purification system and the QIAexpress™ system (Qiagen) useful with (HIS₆) fusion partners. As another example, a fusion domain may be selected so as to facilitate detection of the ActRIIb polypeptides. Examples of such detection domains include the various fluorescent proteins (e.g., GFP) as well as "epitope tags," which are usually short peptide sequences for which a specific antibody is available. Well known epitope tags for which specific monoclonal antibodies are readily available include FLAG, influenza virus haemagglutinin (HA), and c-myc tags. In some cases, the fusion domains have a protease cleavage site, such as for Factor Xa or Thrombin, which allows the relevant protease to partially digest the fusion proteins and thereby liberate the recombinant proteins therefrom. The liberated proteins can then be isolated from the fusion domain by subsequent chromatographic separation. In certain preferred embodiments, an ActRIIb polypeptide is fused with a domain that stabilizes the ActRIIb polypeptide in vivo (a "stabilizer" domain). By "stabilizing" is meant anything that increases serum half life, regardless of whether this is because of decreased destruction, decreased clearance by the kidney, or other pharmacokinetic effect. Fusions with the Fc portion of an immunoglobulin are known to confer desirable pharmacokinetic properties on a wide range of proteins. Constant domains from an immunoglobulin, particularly an IgG heavy chain, may also be used as stabilizing domains. Likewise, fusions to human serum albumin can confer desirable properties. Other types of fusion domains that may be selected include multimerizing (e.g., dimerizing, tetramerizing) domains and functional domains (that confer an additional biological function, such as further stimulation of muscle growth).

As a specific example, the present invention provides a fusion protein comprising a soluble extracellular domain of ActRIIb fused to an Fc domain (Fc portion underlined)(SEQ ID NO:6):

SGRGEAETRECIYYNANWELERTNQSGLERCEGEQDKRLHCYASWRNSSGT
IELVKKGCWLDDFNCYDRQECVATEENPQVYFCCCEGNFCNERFTHLPEAG
GPEVTYEPPPTAPTGGGTHTCPPCPAPELLGGPSVFLFPPKPKDTLMISRTPEV

TCVVVDVSHEDPEVKFNWYVDGVEVHNAKTKPREEQYNSTYRVVSVLTVL
HQDWLNGKEYKCKVSNKALPVPIEKTISKAKGQPREPQVYTLPPSREEMTK
NQVSLTCLVKGFYPSDIAVEWESNGQPENNYKTTPPVLDSDGSFFLYSKLTV
DKSRWQQGNVFSCSVMHEALHNHYTQKSLSLSPGK (SEQ ID NO: 6)

An example of an IgG1 Fc domain is shown below (SEQ ID NO: 7).

THTCPPCPAPELLGGPSVFLFPPKPKDTLMISRTPEVTCVVVD(A)VSHEDPEVKF
NWYVDGVEVHNAKTKPREEQYNSTYRVVSVLTVLHQDWLNGKEYKCK(A)VSNKAL
PVPIEKTISKAKGQPREPQVYTLPPSREEMTKNQVSLTCLVKGFYPSDIAVEWESN
GQPENNYKTTPPVLDSDGPFFLYSKLTVDKSRWQQGNVFSCSVMHEALHN(A)HYT
QKSLSLSPGK*

Optionally, the Fc domain has one or more mutations at residues such as Asp-265, lysine 322, and Asn-434. In certain cases, the mutant Fc domain having one or more of these mutations (e.g., Asp-265 mutation) has reduced ability of binding to the Fcγ receptor relative to a wildtype Fc domain. In other cases, the mutant Fc domain having one or more of these mutations (e.g., Asn-434 mutation) has increased ability of binding to the MHC class I-related Fc-receptor (FcRN) relative to a wildtype Fc domain. Fc domains from IgG2, IgG3 and IgG4 may also be used.

It is understood that different elements of the fusion proteins may be arranged in any manner that is consistent with the desired functionality. For example, an ActRIIb polypeptide may be placed C-terminal to a heterologous domain, or, alternatively, a heterologous domain may be placed C-terminal to an ActRIIb polypeptide. The ActRIIb polypeptide domain and the heterologous domain need not be adjacent in a fusion protein, and additional domains or amino acid sequences may be included C- or N-terminal to either domain or between the domains.

In certain embodiments, the ActRIIb polypeptides of the present invention contain one or more modifications that are capable of stabilizing the ActRIIb polypeptides. For example, such modifications enhance the in vitro half life of the ActRIIb polypeptides, enhance circulatory half life of the ActRIIb polypeptides or reducing proteolytic degradation of the ActRIIb polypeptides. Such stabilizing modifications include, but are not limited to, fusion proteins (including, for example, fusion proteins comprising an ActRIIb polypeptide and a stabilizer domain), modifications of a glycosylation site (including, for example, addition of a glycosylation site to an ActRIIb polypeptide), and modifications of carbohydrate moiety (including, for example, removal of carbohydrate moieties from an ActRIIb polypeptide). As used herein, the term "stabilizer domain" not only refers to a fusion domain (e.g., Fc) as in the case of fusion proteins, but also includes nonproteinaceous modifications such as a carbohydrate moiety, or nonproteinaceous moiety, such as polyethylene glycol.

In certain embodiments, the present invention makes available isolated and/or purified forms of the ActRIIb polypeptides, which are isolated from, or otherwise substantially free of, other proteins. ActRIIb polypeptides will generally be produced by expression from recombinant nucleic acids.

### 3. Nucleic Acids Encoding ActRIIb Polypeptides

The disclosure provides isolated and/or recombinant nucleic acids encoding any of the ActRIIb polypeptides (e.g., full-length and soluble ActRIIb polypeptides), including fragments, functional variants and fusion proteins disclosed herein. For example, SEQ ID NO: 4 encodes the naturally occurring human ActRIIb precursor polypeptide, while SEQ ID NO: 5 encodes the processed extracellular domain of ActRIIb. The subject nucleic acids may be single-stranded or double stranded. Such nucleic acids may be DNA or RNA molecules. These nucleic acids may be used, for example, in methods for making ActRIIb polypeptides or as direct therapeutic agents (e.g., in a gene therapy approach).

In certain aspects, the subject nucleic acids encoding ActRIIb polypeptides are further understood to include nucleic acids that are variants of SEQ ID NO: 4 or 5. Variant nucleotide sequences include sequences that differ by one or more nucleotide substitutions, additions or deletions, such as allelic variants.

The disclosure provides isolated or recombinant nucleic acid sequences that are at least 80%, 85%, 90%, 95%, 97%, 98%, 99% or 100% identical to SEQ ID NOs: 4, 5, or 10. One of ordinary skill in the art will appreciate that nucleic acid sequences complementary to SEQ ID NOs: 4, 5, or 10 and variants of SEQ ID NOs: 4, 5, or 10 are also within the scope of this disclosure. In further disclosures, the nucleic acid sequences can be isolated, recombinant, and/or fused with a heterologous nucleotide sequence, or in a DNA library.

In other disclosures, nucleic acids also include nucleotide sequences, and the ActRIIb polypeptides encoded by such nucleic acids, that hybridize under highly stringent conditions to the nucleotide sequence designated in SEQ ID NOs: 4, 5, or 10, the complement sequence of SEQ ID NOs: 4, 5, or 10, or fragments of any of the foregoing. As discussed above, one of ordinary skill in the art will understand readily that appropriate stringency conditions which promote DNA hybridization can be varied. One of ordinary skill in the art will understand readily that appropriate stringency conditions which promote DNA hybridization can be varied. For example, one could perform the hybridization at 6.0 x sodium chloride/sodium citrate (SSC) at about 45 °C, followed by a wash of 2.0 x SSC at 50 °C. For example, the salt concentration in the wash step can be selected from a low stringency of about 2.0 x SSC at 50 °C to a high stringency of about 0.2 x SSC at 50 °C. In addition, the temperature in the wash step can be increased from low stringency conditions at room temperature, about 22 °C, to high stringency conditions at about 65 °C. Both temperature and salt may be varied, or temperature or salt concentration may be held constant while the other variable is changed. The disclosure provides nucleic acids which hybridize under low stringency conditions of 6 x SSC at room temperature followed by a wash at 2 x SSC at room temperature.

Isolated nucleic acids which differ from the nucleic acids as set forth in SEQ ID NOs: 4, 5, or 10 due to degeneracy in the genetic code are also within the scope of the disclosure. For example, a number of amino acids are designated by more than one triplet. Codons that specify the same amino acid, or synonyms (for example, CAU and CAC are synonyms for histidine) may result in "silent" mutations which do not affect the amino acid sequence of the protein. However, it is expected that DNA sequence polymorphisms that do lead to changes in the amino acid sequences of the subject proteins will exist among mammalian cells. One skilled in the art will appreciate that these variations in one or more nucleotides (up to about 3-5% of the nucleotides) of the nucleic acids encoding a particular protein may exist among individuals of a given species due to natural allelic variation. Any and all such nucleotide variations and resulting amino acid polymorphisms are within the scope of this disclosure.

In certain embodiments, the recombinant nucleic acids of the invention may be operably linked to one or more regulatory nucleotide sequences in an expression construct. Regulatory nucleotide sequences will generally be appropriate to the host cell used for expression. Numerous types of appropriate expression vectors and suitable regulatory sequences are known in the art for a variety of host cells. Typically, said one or more regulatory nucleotide sequences may include, but are not limited to, promoter sequences, leader or signal sequences, ribosomal binding sites, transcriptional start and termination sequences, translational start and termination sequences, and enhancer or activator sequences. Constitutive or inducible promoters as known in the art are contemplated by the invention. The promoters may be either naturally occurring promoters, or hybrid promoters that combine elements of more than one promoter. An expression construct may be present in a cell on an episome, such as a plasmid, or the expression construct may be inserted in a chromosome. In a preferred embodiment, the expression vector contains a selectable marker gene to allow the selection of transformed host cells. Selectable marker genes are well known in the art and will vary with the host cell used.

In certain aspects of the invention, the subject nucleic acid is provided in an expression vector comprising a nucleotide sequence encoding an ActRIIb polypeptide and operably linked to at least one regulatory sequence. Regulatory sequences are art-recognized and are selected to direct expression of the ActRIIb polypeptide. Accordingly, the term regulatory sequence includes promoters, enhancers, and other expression control elements. Exemplary regulatory sequences are described in Goeddel; Gene Expression Technology: Methods in Enzymology, Academic Press, San Diego, CA (1990). For instance, any of a wide variety of expression control sequences that control the expression of a DNA sequence when operatively linked to it may be used in these vectors to express DNA sequences encoding an ActRIIb polypeptide. Such useful expression control sequences, include, for example, the early and late promoters of SV40, tet promoter, adenovirus or cytomegalovirus immediate early promoter, RSV promoters, the lac system, the trp system, the TAC or TRC system, T7 promoter whose expression is directed by T7 RNA polymerase, the major operator and promoter regions of phage lambda , the control regions for fd coat protein, the promoter for 3-phosphoglycerate kinase or other glycolytic enzymes, the promoters of acid phosphatase, e.g., Pho5, the promoters of the yeast α-mating factors, the polyhedron promoter of the baculovirus system and other sequences known to control the expression of genes of prokaryotic or eukaryotic cells or their viruses, and various combinations thereof. It should be understood that the design of the expression vector may depend on such factors as the choice of the host cell to be transformed and/or the type of protein desired to be expressed. Moreover, the vector's copy number, the ability to control that copy number and the expression of any other protein encoded by the vector, such as antibiotic markers, should also be considered.

A recombinant nucleic acid of the invention can be produced by ligating the cloned gene, or a portion thereof, into a vector suitable for expression in either prokaryotic cells, eukaryotic cells (yeast, avian, insect or mammalian), or both. Expression vehicles for production of a recombinant ActRIIb polypeptide include plasmids and other vectors. For instance, suitable vectors include plasmids of the types: pBR322-derived plasmids, pEMBL-derived plasmids, pEX-derived plasmids, pBTac-derived plasmids and pUC-derived plasmids for expression in prokaryotic cells, such as *E. coli.*

Some mammalian expression vectors contain both prokaryotic sequences to facilitate the propagation of the vector in bacteria, and one or more eukaryotic transcription units that are expressed in eukaryotic cells. The pcDNAI/amp, pcDNAI/neo, pRc/CMV, pSV2gpt, pSV2neo, pSV2-dhfr, pTk2, pRSVneo, pMSG, pSVT7, pko-neo and pHyg derived vectors are examples of mammalian expression vectors suitable for transfection of eukaryotic cells. Some of these vectors are modified with sequences from bacterial plasmids, such as pBR322, to facilitate replication and drug resistance selection in both prokaryotic and eukaryotic cells. Alternatively, derivatives of viruses such as the bovine papilloma virus (BPV-1), or Epstein-Barr virus (pHEBo, pREP-derived and p205) can be used for transient expression of proteins in eukaryotic cells. Examples of other viral (including retroviral) expression systems can be found below in the description of gene therapy delivery systems. The various methods employed in the preparation of the plasmids and in transformation of host organisms are well known in the art. For other suitable expression systems for both prokaryotic and eukaryotic cells, as well as general recombinant procedures, see Molecular Cloning A Laboratory Manual, 3rd Ed., ed. by Sambrook, Fritsch and Maniatis (Cold Spring Harbor Laboratory Press, 2001). In some instances, it may be desirable to express the recombinant polypeptides by the use of a baculovirus expression system. Examples of such baculovirus expression systems include pVL-derived vectors (such as pVL1392, pVL1393 and pVL941), pAcUW-derived vectors (such as pAcUW1), and pBlueBac-derived vectors (such as the β-gal containing pBlueBac III).

In a preferred embodiment, a vector will be designed for production of the subject ActRIIb polypeptides in CHO cells, such as a Pcmv-Script vector (Stratagene, La Jolla, Calif.), pcDNA4 vectors (Invitrogen, Carlsbad, Calif.) and pCI-neo vectors (Promega, Madison, Wise.). As will be apparent, the subject gene constructs can be used to cause expression of the subject ActRIIb polypeptides in cells propagated in culture, e.g., to produce proteins, including fusion proteins or variant proteins, for purification.

This disclosure also pertains to a host cell transfected with a recombinant gene including a coding sequence (e.g., SEQ ID NOs: 4, 5, or 10) for one or more of the subject ActRIIb polypeptides. The host cell may be any prokaryotic or eukaryotic cell. For example, an ActRIIb polypeptide of the invention may be expressed in bacterial cells such as *E. coli*, insect cells (e.g., using a baculovirus expression system), yeast, or mammalian cells. Other suitable host cells are known to those skilled in the art.

Accordingly, the present disclosure further pertains to methods of producing the subject ActRIIb polypeptides. For example, a host cell transfected with an expression vector encoding an ActRIIb polypeptide can be cultured under appropriate conditions to allow expression of the ActRIIb polypeptide to occur. The ActRIIb polypeptide may be secreted and isolated from a mixture of cells and medium containing the ActRIIb polypeptide. Alternatively, the ActRIIb polypeptide may be retained cytoplasmically or in a membrane fraction and the cells harvested, lysed and the protein isolated. A cell culture includes host cells, media and other byproducts. Suitable media for cell culture are well known in the art. The subject ActRIIb polypeptides can be isolated from cell culture medium, host cells, or both, using techniques known in the art for purifying proteins, including ion-exchange chromatography, gel filtration chromatography, ultrafiltration, electrophoresis, immunoaffinity purification with antibodies specific for particular epitopes of the ActRIIb polypeptides and affinity purification with an agent that binds to a domain fused to the ActRIIb polypeptide (e.g., a protein A column may be used to purify an ActRIIb-Fc fusion). In a preferred embodiment, the ActRIIb polypeptide is a fusion protein containing a domain which facilitates its purification. In a preferred embodiment, purification is achieved by a series of column chromatography steps, including, for example, three or more of the following, in any order: protein A chromatography, Q sepharose chromatography, phenylsepharose chromatography, size exclusion chromatography, and cation exchange chromatography. The purification could be completed with viral filtration and buffer exchange.

In another embodiment, a fusion gene coding for a purification leader sequence, such as a poly-(His)/enterokinase cleavage site sequence at the N-terminus of the desired portion of the recombinant ActRIIb polypeptide, can allow purification of the expressed fusion protein by affinity chromatography using a Ni²⁺ metal resin. The purification leader sequence can then be subsequently removed by treatment with enterokinase to provide the purified ActRIIb polypeptide (e.g., see Hochuli et al., (1987) J. Chromatography 411:177; and Janknecht et al., PNAS USA 88:8972).

Techniques for making fusion genes are well known. Essentially, the joining of various DNA fragments coding for different polypeptide sequences is performed in accordance with conventional techniques, employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. In another embodiment, the fusion gene can be synthesized by conventional techniques including automated DNA synthesizers. Alternatively, PCR amplification of gene fragments can be carried out using anchor primers which give rise to complementary overhangs between two consecutive gene fragments which can subsequently be annealed to generate a chimeric gene sequence (see, for example, Current Protocols in Molecular Biology, eds. Ausubel et al., John Wiley & Sons: 1992).

### 4. Alternative ActRIIb Antagonists

As demonstrated herein, an ActRIIb polypeptide is effective to increase reticulocyte levels in vivo, an effect which, over a longer time period leads to increased hematocrit levels in certain species, and is likely to do so in humans. Thus, in some embodiments, ActRIIb antagonists of the disclosure may be used increase red blood cell levels in vivo. Although soluble ActRIIb polypeptides, and particularly ActRIIb-Fc, are preferred antagonists, and although such antagonists may affect red blood cell levels through a mechanism other than activin antagonism (e.g., activin inhibition may be an indicator of the tendency of an agent to inhibit the activities of a spectrum of molecules, including, perhaps, other members of the TGF-beta superfamily, and such collective inhibition may lead to the desired effect on hematopoiesis), other types of ActRIIb antagonists are expected to be useful, including anti-ActRIIb antibodies, antisense, RNAi or ribozyme nucleic acids that inhibit the production of ActRIIb, and other inhibitors of ActRIIb, particularly those that disrupt ActRIIb binding.

An antibody that is specifically reactive with an ActRIIb polypeptide (e.g., a soluble ActRIIbpolypeptide) and which either binds competitively to ligand with the ActRIIb polypeptide or otherwise inhibits ActRIIb -mediated signaling may be used as an antagonist of ActRIIb polypeptide activities.

By using immunogens derived from an ActRIIb polypeptide, anti-protein/anti-peptide antisera or monoclonal antibodies can be made by standard protocols (see, for example, Antibodies: A Laboratory Manual ed. by Harlow and Lane (Cold Spring Harbor Press: 1988)). A mammal, such as a mouse, a hamster or rabbit can be immunized with an immunogenic form of the ActRIIb polypeptide, an antigenic fragment which is capable of eliciting an antibody response, or a fusion protein. Techniques for conferring immunogenicity on a protein or peptide include conjugation to carriers or other techniques well known in the art. An immunogenic portion of an ActRIIb polypeptide can be administered in the presence of adjuvant. The progress of immunization can be monitored by detection of antibody titers in plasma or serum. Standard ELISA or other immunoassays can be used with the immunogen as antigen to assess the levels of antibodies.

Following immunization of an animal with an antigenic preparation of an ActRIIb polypeptide, antisera can be obtained and, if desired, polyclonal antibodies can be isolated from the serum. To produce monoclonal antibodies, antibody-producing cells (lymphocytes) can be harvested from an immunized animal and fused by standard somatic cell fusion procedures with immortalizing cells such as myeloma cells to yield hybridoma cells. Such techniques are well known in the art, and include, for example, the hybridoma technique (originally developed by Kohler and Milstein, (1975) Nature, 256: 495-497), the human B cell hybridoma technique (Kozbar et al., (1983) Immunology Today, 4: 72), and the EBV-hybridoma technique to produce human monoclonal antibodies (Cole et al., (1985) Monoclonal Antibodies and Cancer Therapy, Alan R. Liss, Inc. pp. 77-96). Hybridoma cells can be screened immunochemically for production of antibodies specifically reactive with an ActRIIb polypeptide and monoclonal antibodies isolated from a culture comprising such hybridoma cells.

The term "antibody" as used herein is intended to include whole antibodies, e.g., of any isotype (IgG, IgA, IgM, IgE, etc), and includes fragments or domains of immunoglobulins which are reactive with a selected antigen. Antibodies can be fragmented using conventional techniques and the fragments screened for utility and/or interaction with a specific epitope of interest. Thus, the term includes segments of proteolytically-cleaved or recombinantly-prepared portions of an antibody molecule that are capable of selectively reacting with a certain protein. Non-limiting examples of such proteolytic and/or recombinant fragments include Fab, F(ab')2, Fab', Fv, and single chain antibodies (scFv) containing a V[L] and/or V[H] domain joined by a peptide linker. The scFv's may be covalently or non-covalently linked to form antibodies having two or more binding sites. The term antibody also includes polyclonal, monoclonal, or other purified preparations of antibodies and recombinant antibodies. The term "recombinant antibody", means an antibody, or antigen binding domain of an immunoglobulin, expressed from a nucleic acid that has been constructed using the techniques of molecular biology, such as a humanized antibody or a fully human antibody developed from a single chain antibody. Single domain and single chain antibodies are also included within the term "recombinant antibody".

In certain cases, an antibody of the disclosure is a monoclonal antibody, and in certain cases, the disclosure makes available methods for generating novel antibodies. For example, a method for generating a monoclonal antibody that binds specifically to an ActRIIb polypeptide may comprise administering to a mouse an amount of an immunogenic composition comprising the antigen polypeptide effective to stimulate a detectable immune response, obtaining antibody-producing cells (e.g., cells from the spleen) from the mouse and fusing the antibody-producing cells with myeloma cells to obtain antibody-producing hybridomas, and testing the antibody-producing hybridomas to identify a hybridoma that produces a monocolonal antibody that binds specifically to the antigen. Once obtained, a hybridoma can be propagated in a cell culture, optionally in culture conditions where the hybridoma-derived cells produce the monoclonal antibody that binds specifically to the antigen. The monoclonal antibody may be purified from the cell culture.

The adjective "specifically reactive with" as used in reference to an antibody is intended to mean, as is generally understood in the art, that the antibody is sufficiently selective between the antigen of interest (e.g., an ActRIIb polypeptide) and other antigens that are not of interest that the antibody is useful for, at minimum, detecting the presence of the antigen of interest in a particular type of biological sample. In certain methods employing the antibody, such as therapeutic applications, a higher degree of specificity in binding may be desirable. Monoclonal antibodies generally have a greater tendency (as compared to polyclonal antibodies) to discriminate effectively between the desired antigens and cross-reacting polypeptides. One characteristic that influences the specificity of an antibody: antigen interaction is the affinity of the antibody for the antigen. Although the desired specificity may be reached with a range of different affinities, generally preferred antibodies will have an affinity (a dissociation constant) of about 10⁻⁶, 10⁻⁷, 10⁻⁸, 10⁻⁹ M or less.

In addition, the techniques used to screen antibodies in order to identify a desirable antibody may influence the properties of the antibody obtained. For example, if an antibody is to be used for binding an antigen in solution, it may be desirable to test solution binding. A variety of different techniques are available for testing interaction between antibodies and antigens to identify particularly desirable antibodies. Such techniques include ELISAs, surface plasmon resonance binding assays (e.g., the Biacore™ binding assay, Biacore AB, Uppsala, Sweden), sandwich assays (e.g., the paramagnetic bead system of IGEN International, Inc., Gaithersburg, Maryland), western blots, immunoprecipitation assays, and immunohistochemistry.

Examples of categories of nucleic acid compounds that are ActRIIb antagonists include antisense nucleic acids, RNAi constructs and catalytic nucleic acid constructs. A nucleic acid compound may be single or double stranded. A double stranded compound may also include regions of overhang or non-complementarity, where one or the other of the strands is single stranded. A single stranded compound may include regions of self-complementarity, meaning that the compound forms a so-called "hairpin" or "stem-loop" structure, with a region of double helical structure. A nucleic acid compound may comprise a nucleotide sequence that is complementary to a region consisting of no more than 1000, no more than 500, no more than 250, no more than 100, or no more than 50, 35, 25, 22, 20, 18 or 15 nucleotides of the full-length ActRIIb nucleic acid sequence. The region of complementarity will preferably be at least 8 nucleotides, and optionally about 18 to 35 nucleotides. A region of complementarity may fall within an intron, a coding sequence or a noncoding sequence of the target transcript, such as the coding sequence portion. Generally, a nucleic acid compound will have a length of about 8 to about 500 nucleotides or base pairs in length, and optionally the length will be about 14 to about 50 nucleotides. A nucleic acid may be a DNA (particularly for use as an antisense), RNA or RNA:DNA hybrid. Any one strand may include a mixture of DNA and RNA, as well as modified forms that cannot readily be classified as either DNA or RNA. Likewise, a double stranded compound may be DNA:DNA, DNA:RNA or RNA:RNA, and any one strand may also include a mixture of DNA and RNA, as well as modified forms that cannot readily be classified as either DNA or RNA. A nucleic acid compound may include any of a variety of modifications, including one or modifications to the backbone (the sugar-phosphate portion in a natural nucleic acid, including internucleotide linkages) or the base portion (the purine or pyrimidine portion of a natural nucleic acid). An antisense nucleic acid compound will preferably have a length of about 15 to about 30 nucleotides and will often contain one or more modifications to improve characteristics such as stability in the serum, in a cell or in a place where the compound is likely to be delivered, such as the stomach in the case of orally delivered compounds and the lung for inhaled compounds. In the case of an RNAi construct, the strand complementary to the target transcript will generally be RNA or modifications thereof. The other strand may be RNA, DNA or any other variation. The duplex portion of double stranded or single stranded "hairpin" RNAi construct will generally have a length of 18 to 40 nucleotides in length and optionally about 21 to 23 nucleotides in length, so long as it serves as a Dicer substrate. Catalytic or enzymatic nucleic acids may be ribozymes or DNA enzymes and may also contain modified forms. Nucleic acid compounds may inhibit expression of the target by about 50%, 75%, 90% or more when contacted with cells under physiological conditions and at a concentration where a nonsense or sense control has little or no effect. Preferred concentrations for testing the effect of nucleic acid compounds are 1, 5 and 10 micromolar. Nucleic acid compounds may also be tested for effects on, for example, red blood cell levels.

In the disclosure, alternative antagonists with properties that are similar to ActRIIb antagonists may be used. An antagonist may be a follistatin polypeptide that antagonizes activin bioactivity and/or binds to activin and/or myostatin. The term "follistatin polypeptide" includes polypeptides comprising any naturally occurring polypeptide of follistatin as well as any variants thereof (including mutants, fragments, fusions, and peptidomimetic forms) that retain a useful activity, and further includes any functional monomer or multimer of follistatin. Variants of follistatin polypeptides that retain activin binding properties can be identified based on previous studies involving follistatin and activin interactions. For example, WO2008/030367 discloses specific follistatin domains ("FSDs") that are shown to be important for activin binding. As shown below in SEQ ID NOs: 18-20, the N-terminus follistatin domain ("FSND" SEQ ID NO: 18), FSD2 (SEQ ID NO: 19), and to a lesser extent FSD1 (SEQ ID NO: 20) represent exemplary domains within follistatin important for activin binding. In addition, methods for making and testing libraries of polypeptides are described above in the context of ActRIIb polypeptides and such methods also pertain to making and testing variants of follistatin. Additionally, forms of follistatin that bind myostatin preferentially (with reduced activin binding) are also known and may be used as antagonists herein that may exhibit properties similar to those of ActRIIb antagonists; such follistatin forms may be found in, for example, WO/2005/100563 and WO/2008/030367). Follistatin polypeptides include polypeptides derived from the sequence of any known follistatin having a sequence at least about 80% identical to the sequence of a follistatin polypeptide, and optionally at least 85%, 90%, 95%, 97%, 99% or greater identity. Examples of follistatin polypeptides include the mature follistatin polypeptide or shorter isoforms or other variants of the human follistatin precursor polypeptide (SEQ ID NO: 16) as described, for example, in WO2005/025601.

The human follistatin precursor polypeptide isoform FST344 is as follows:

MVRARHQPGGLCLLLLLLCQFMEDRSAQAGNCWLRQAKNGRCQV
LYKTELSKEECCSTGRLSTSWTEEDVNDNTLFKWMIFNGGAPNC
IPCKETCENVDCGPGKKCRMNKKNKPRCVCAPDCSNITWKGPVC
GLDGKTYRNECALLKARCKEQPELEVQYQGRCKKTCRDVFCPGS
STCVVDQTNNAYCVTCNRICPEPASSEQYLCGNDGVTYSSACHL
RKATCLLGRSIGLAYEGKCIKAKSCEDIQCTGGKKCLWDFKVGR
GRCSLCDELCPDSKSDEPVCASDNATYASECAMKEAACSSGVLL
EVKHGSCNSISEDTEEEEEDEDQDYSFPISSILEW (SEQ ID
NO: 16; NP_037541.1 FOLLISTATIN ISOFORM FST344)

The signal peptide is single underlined; the last 27 residues in bold represent additional amino acids as compared to a shorter follistatin isoform FST317 (NP_006341) below.

The human follistatin precursor polypeptide isoform FST317 is as follows: 
MVRARHQPGGLCLLLLLLCQFMEDRSAQAGNCWLRQAKNGRCQV
LYKTELSKEECCSTGRLSTSWTEEDVNDNTLFKWMIFNGGAPNC
IPCKETCENVDCGPGKKCRMNKKNKPRCVCAPDCSNITWKGPVC
GLDGKTYRNECALLKARCKEQPELEVQYQGRCKKTCRDVFCPGS
STCVVDQTNNAYCVTCNRICPEPASSEQYLCGNDGVTYSSACHL
RKATCLLGRSIGLAYEGKCIKAKSCEDIQCTGGKKCLWDFKVGR
GRCSLCDELCPDSKSDEPVCASDNATYASECAMKEAACSSGVLL
EVKHSGSCN (SEQ ID NO: 17)
 The signal peptide is single underlined.

N-terminus follistatin domain (FSND) sequence is as follows:

GNCWLRQAKNGRCQVLYKTELSKEECCSTGRLSTSWTEEDVNDN
TLFKWMIFNGGAPNCIPCK (SEQ ID NO: 18; FSND)

The FSD1 and FSD2 sequences are as follows:
ETCENVDCGPGKKCRMNKKNKPRCV (SEQ ID NO: 19; FSD1)
KTCRDVFCPGSSTCVVDQTNNAYCVT (SEQ ID NO: 20; FSD2)

In other disclosures, an antagonist similar to an ActRIIb antagonist may be a follistatin-like related gene (FLRG) that antagonizes activin bioactivity and/or binds to activin. The term "FLRG polypeptide" includes polypeptides comprising any naturally occurring polypeptide of FLRG as well as any variants thereof (including mutants, fragments, fusions, and peptidomimetic forms) that retain a useful activity. Variants of FLRG polypeptides that retain activin or myostatin binding properties can be identified using routine methods to assay FLRG and activin or myostatin interactions. See, for example, US 6,537,966. In addition, methods for making and testing libraries of polypeptides are described above in the context of ActRIIb polypeptides and such methods also pertain to making and testing variants of FLRG. FLRG polypeptides include polypeptides derived from the sequence of any known FLRG having a sequence at least about 80% identical to the sequence of an FLRG polypeptide, and optionally at least 85%, 90%, 95%, 97%, 99% or greater identity.

The human FLRG precursor polypeptide is as follows: 
MRPGAPGPLWPLPWGALAWAVGFVSSMGSGNPAPGGVCWLQQGQ
EATCSLVLQTDVTRAECCASGNIDTAWSNLTHPGNKINLLGFLG
 
LVHCLPCKDSCDGVECGPGKACRMLGGRPRCECAPDCSGLPARL
QVCGSDGATYRDECELRAARCRGHPDLSVMYRGRCRKSCEHVVC
PRPQSCVVDQTGSAHCVVCRAAPCVPSSPGQELCGNNNVTYISS
CHMRQATCFLGRSIGVRHAGSCAGTPEEPPGGESAEEEENFV
(SEQ ID NO: 21; NP_005851)
 The signal peptide is single underlined.

In certain disclosures, functional variants or modified forms of the follistatin polypeptides and FLRG polypeptides include fusion protein having at least a portion of the follistatin polypeptides or FLRG polypeptides and one or more fusion domains, such as, for example, domains that facilitate isolation, detection, stabilization or multimerization of the polypeptide. Suitable fusion domains are discussed in detail above with reference to the ActRIIb polypeptides. In one disclosure, an antagonist is a fusion protein comprising a ligand binding (e.g. activin binding) portion of a follistaton polypeptide fused to an Fc domain. In another disclosure, an antagonist is a fusion protein comprising a ligand binding (e.g. activin binding) portion of an FLRG polypeptide fused to an Fc domain. Follistatin and FLRG have been shown in the literature, and by the applicants with respect to FLRG, to have affinities for Activin A in the picomolar range, indicating that these agents will inhibit activin A signaling to a similar degree as ActRIIb-Fc.

### 5. Screening Assays

In certain aspects, the present disclosure relates to the use of ActRIIb polypeptides and activin polypeptides to identify compounds (agents) which are agonist or antagonists of the ActRIIb signaling pathway. Compounds identified through this screening can be tested to assess their ability to modulate red blood cell, hemoglobin and/or reticulocyte levels in vivo or in vitro. These compounds can be tested, for example, in animal models.

There are numerous approaches to screening for therapeutic agents for increasing red blood cell or hemoglobin levels by targeting activin, myostatin (or other ligands) and ActRIIb signaling. In certain disclosures, high-throughput screening of compounds can be carried out to identify agents that perturb activin/myostatin or ActRIIb-mediated effects on a selected cell line. In certain disclosures, the assay is carried out to screen and identify compounds that specifically inhibit or reduce binding of an ActRIIb polypeptide to activin, myostatin or other ligands. Alternatively, the assay can be used to identify compounds that enhance binding of an ActRIIb polypeptide to activin, myostatin or other ligands. In a further disclosure, the compounds can be identified by their ability to interact with an activin or ActRIIb polypeptide.

A variety of assay formats will suffice and, in light of the present disclosure, those not expressly described herein will nevertheless be comprehended by one of ordinary skill in the art. As described herein, the test compounds (agents) of the disclosure may be created by any combinatorial chemical method. Alternatively, the subject compounds may be naturally occurring biomolecules synthesized in vivo or in vitro. Compounds (agents) to be tested for their ability to act as modulators of tissue growth can be produced, for example, by bacteria, yeast, plants or other organisms (e.g., natural products), produced chemically (e.g., small molecules, including peptidomimetics), or produced recombinantly. Test compounds contemplated by the present disclosure include non-peptidyl organic molecules, peptides, polypeptides, peptidomimetics, sugars, hormones, and nucleic acid molecules. In a specific disclosure, the test agent is a small organic molecule having a molecular weight of less than about 2,000 Daltons.

The test compounds of the disclosure can be provided as single, discrete entities, or provided in libraries of greater complexity, such as made by combinatorial chemistry. These libraries can comprise, for example, alcohols, alkyl halides, amines, amides, esters, aldehydes, ethers and other classes of organic compounds. Presentation of test compounds to the test system can be in either an isolated form or as mixtures of compounds, especially in initial screening steps. Optionally, the compounds may be optionally derivatized with other compounds and have derivatizing groups that facilitate isolation of the compounds. Non-limiting examples of derivatizing groups include biotin, fluorescein, digoxygenin, green fluorescent protein, isotopes, polyhistidine, magnetic beads, glutathione S transferase (GST), photoactivatible crosslinkers or any combinations thereof.

In many drug screening programs which test libraries of compounds and natural extracts, high throughput assays are desirable in order to maximize the number of compounds surveyed in a given period of time. Assays which are performed in cell-free systems, such as may be derived with purified or semipurified proteins, are often preferred as "primary" screens in that they can be generated to permit rapid development and relatively easy detection of an alteration in a molecular target which is mediated by a test compound. Moreover, the effects of cellular toxicity or bioavailability of the test compound can be generally ignored in the in vitro system, the assay instead being focused primarily on the effect of the drug on the molecular target as may be manifest in an alteration of binding affinity between an ActRIIb polypeptide and a ligand such as activin or myostatin.

Merely to illustrate, in an exemplary screening assay of the present disclosure, the compound of interest is contacted with an isolated and purified ActRIIb polypeptide which is ordinarily capable of binding to a ligand such as activin or myostatin. To the mixture of the compound and ActRIIb polypeptide is then added a composition containing an ActRIIb ligand. Detection and quantification of ActRIIb/ligand (e.g., activin, myostatin) complexes provides a means for determining the compound's efficacy at inhibiting (or potentiating) complex formation between the ActRIIb polypeptide and a ligand. The efficacy of the compound can be assessed by generating dose response curves from data obtained using various concentrations of the test compound. Moreover, a control assay can also be performed to provide a baseline for comparison. For example, in a control assay, isolated and purified activin is added to a composition containing the ActRIIb polypeptide, and the formation of ActRIIb/ligand complex is quantitated in the absence of the test compound. It will be understood that, in general, the order in which the reactants may be admixed can be varied, and can be admixed simultaneously. Moreover, in place of purified proteins, cellular extracts and lysates may be used to render a suitable cell-free assay system.

Complex formation between the ActRIIb polypeptide and activin may be detected by a variety of techniques. For instance, modulation of the formation of complexes can be quantitated using, for example, detectably labeled proteins such as radiolabeled (e.g., ³²P, ³⁵S, ¹⁴C or ³H), fluorescently labeled (e.g., FITC), or enzymatically labeled ActRIIb polypeptide or ligand, by immunoassay, or by chromatographic detection.

The present disclosure contemplates the use of fluorescence polarization assays and fluorescence resonance energy transfer (FRET) assays in measuring, either directly or indirectly, the degree of interaction between an ActRIIb polypeptide and its binding protein. Further, other modes of detection, such as those based on optical waveguides (PCT Publication WO 96/26432 and U.S. Pat. No. 5,677,196), surface plasmon resonance (SPR), surface charge sensors, and surface force sensors, are compatible with the present disclosure.

Moreover, the present disclosure contemplates the use of an interaction trap assay, also known as the "two hybrid assay," for identifying agents that disrupt or potentiate interaction between an ActRIIb polypeptide and its binding protein. See for example, U.S. Pat. No. 5,283,317; Zervos et al. (1993) Cell 72:223-232; Madura et al. (1993) J Biol Chem 268:12046-12054; Bartel et al. (1993) Biotechniques 14:920-924; and Iwabuchi et al. (1993) Oncogene 8:1693-1696). The present disclosure contemplates the use of reverse two hybrid systems to identify compounds (e.g., small molecules or peptides) that dissociate interactions between an ActRIIb polypeptide and its binding protein. See for example, Vidal and Legrain, (1999) Nucleic Acids Res 27:919-29; Vidal and Legrain, (1999) Trends Biotechnol 17:374-81; and U.S. Pat. Nos. 5,525,490; 5,955,280; and 5,965,368.

In certain cases, the subject compounds are identified by their ability to interact with an ActRIIb or ligand polypeptide of the disclosure. The interaction between the compound and the ActRIIb or ligand polypeptide may be covalent or non-covalent. For example, such interaction can be identified at the protein level using in vitro biochemical methods, including photo-crosslinking, radiolabeled ligand binding, and affinity chromatography (Jakoby WB et al., 1974, Methods in Enzymology 46: 1). In certain cases, the compounds may be screened in a mechanism based assay, such as an assay to detect compounds which bind to a ligand or ActRIIb polypeptide. This may include a solid phase or fluid phase binding event. Alternatively, the gene encoding an ActRIIb polypeptide can be transfected with a reporter system (e.g., β-galactosidase, luciferase, or green fluorescent protein) into a cell and screened against the library optionally by a high throughput screening or with individual members of the library. Other mechanism based binding assays may be used, for example, binding assays which detect changes in free energy. Binding assays can be performed with the target fixed to a well, bead or chip or captured by an immobilized antibody or resolved by capillary electrophoresis. The bound compounds may be detected usually using colorimetric or fluorescence or surface plasmon resonance.

### 6. Exemplary Therapeutic Uses

In certain embodiments, compositions for use comprising ActRIIb antagonists (e.g., ActRIIb polypeptides) of the present invention can be used to increase red blood cell levels in mammals such as rodents and primates, and particularly human patients. In certain embodiments, the present invention provides compositions for use in methods of treating or preventing anemia in an individual in need thereof by administering to the individual a therapeutically effective amount of an ActRIIb antagonist, such as an ActRIIb polypeptide. In certain embodiments, the present invention provides compositions for use in methods of promoting red blood cell formation in an individual by administering to the individual a therapeutically effective amount of an ActRIIb antagonist, particularly an ActRIIb polypeptide. These methods may be used for therapeutic and prophylactic treatments of mammals, and particularly humans.

As used herein, a therapeutic that "prevents" a disorder or condition refers to a compound that, in a statistical sample, reduces the occurrence of the disorder or condition in the treated sample relative to an untreated control sample, or delays the onset or reduces the severity of one or more symptoms of the disorder or condition relative to the untreated control sample. The term "treating" as used herein includes prophylaxis of the named condition or amelioration or elimination of the condition once it has been established. In either case, prevention or treatment may be discerned in the diagnosis provided by a physician or other health care provider and the intended result of administration of the therapeutic agent.

As shown herein, ActRIIb antagonists may be used to increase red blood cell, hemoglobin or reticulocyte levels in healthy individuals, and such antagonists may be used in selected patient populations. Examples of appropriate patient populations include those with undesirably low red blood cell or hemoglobin levels, such as patients having an anemia, and those that are at risk for developing undesirably low red blood cell or hemoglobin levels, such as those patients that are about to undergo major surgery or other procedures that may result in substantial blood loss. In one embodiment, a patient with adequate red blood cell levels is treated with an ActRIIb antagonist to increase red blood cell levels, and then blood is drawn and stored for later use in transfusions.

As described in the examples, ActRIIb antagonists may stimulate red blood cell production by activation of splenic erythropoiesis. This novel mechanism indicates that these antagonists are likely to work synergistically with other anemia treatments, such as erythropoietin agonists (e.g., Epogen, Procrit, Aranesp, Epo mimics, Epo receptor agonists, etc.).

ActRIIb antagonists disclosed herein, and particularly ActRIIb-Fc proteins, may be used to increase red blood cell levels in patients having an anemia. When observing hemoglobin levels in humans, a level of less than normal for the appropriate age and gender category may be indicative of anemia, although individual variations are taken into account. For example, a hemoglobin level of 12 g/dl is generally considered the lower limit of normal in the general adult population. Potential causes include blood-loss, nutritional deficits, medication reaction, various problems with the bone marrow and many diseases. More particularly, anemia has been associated with a variety of disorders that include, for example, chronic renal failure, myelodysplastic syndrome, myelofibrosis, rheumatoid arthritis, bone marrow transplantation. Anemia may also be associated with the following conditions: solid tumors (e.g. breast cancer, lung cancer, colon cancer); tumors of the lymphatic system (e.g. chronic lymphocyte leukemia, non-Hodgkins and Hodgkins lymphomas); tumors of the hematopoietic system (eg. leukemia, myelodysplastic syndrome, multiple myeloma); radiation therapy; chemotherapy (e.g. platinum containing regimens); inflammatory and autoimmune diseases, including, but not limited to, rheumatoid arthritis, other inflammatory arthritides, systemic lupus erythematosis (SLE), acute or chronic skin diseases (e.g. psoriasis), inflammatory bowel disease (e.g. Crohn's disease and ulcerative colitis); acute or chronic renal disease or failure including idiopathic or congenital conditions; acute or chronic liver disease; acute or chronic bleeding; situations where transfusion of red blood cells is not possible due to patient allo- or auto-antibodies and/or for religious reasons (e.g. some Jehovah's Witnesses); infections (e.g. malaria, osteomyelitis); hemoglobinopathies, including, for example, sickle cell disease, thalassemias; drug use or abuse, e.g. alcohol misuse; pediatric patients with anemia from any cause to avoid transfusion; and elderly patients or patients with underlying cardiopulmonary disease with anemia who cannot receive transfusions due to concerns about circulatory overload.

ActRIIb antagonists (e.g., ActRIIb polypeptides) would be appropriate for treating anemias of hypoproliferative bone marrrow, which are typically associated with little change in RBC morphology. Hypoproliferative anemias include: 1) anemia of chronic disease, 2) anemia of kidney disease, and 3) anemia associated with hypometabolic states. In each of these types, endogenous erythropoietin levels are *inappropriately low* for the degree of anemia observed. Other hypoproliferative anemias include: 4) early-stage iron-deficient anemia, and 5) anemia caused by damage to the bone marrow. In these types, endogenous erythropoietin levels are *appropriately elevated* for the degree of anemia observed.

The most common type is anemia of chronic disease, which encompasses inflammation, infection, tissue injury, and conditions such as cancer, and is distinguished by both low erythropoietin levels and an *inadequate response* to erythropoietin in the bone marrow (Adamson, 2008, Harrison's Principles of Internal Medicine, 17th ed.; McGraw Hill, New York, pp 628-634). Many factors can contribute to cancer-related anemia. Some are associated with the disease process itself and the generation of inflamatory cytokines such as interleukin-1, interferon-gamma, and tumor necrosis factor (Bron et al., 2001, Semin Oncol 28(Suppl 8):1-6). Among its effects, inflammation induces the key iron-regulatory peptide hepcidin, thereby inhibiting iron export from macrophages and generally limiting iron availability for erythropoiesis (Ganz, 2007, J Am Soc Nephrol 18:394-400). Blood loss through various routes can also contribute to cancer-related anemia. The prevalence of anemia due to cancer progression varies with cancer type, ranging from 5% in prostate cancer up to 90% in multiple myeloma. Cancer-related anemia has profound consequences for patients, including fatigue and reduced quality of life, reduced treatment efficacy, and increased mortality.

Chronic kidney disease is associated with hypoproliferative anemia that varies in severity with the degree of renal impairment. Such anemia is primarily due to inadequate *production* of erythropoietin and reduced survival of red blood cells. Chronic kidney disease usually proceeds gradually over a period of years or decades to end-stage (Stage-5) disease, at which point dialysis or kidney transplantation is required for patient survival. Anemia often develops early in this process and worsens as disease progresses. The clinical consequences of anemia of kidney disease are well-documented and include development of left ventricular hypertrophy, impaired cognitive function, reduced quality of life, and altered immune function (Levin et al., 1999, Am J Kidney Dis 27:347-354; Nissenson, 1992, Am J Kidney Dis 20(Suppl 1):21-24; Revicki et al., 1995, Am J Kidney Dis 25:548-554; Gafter et al., 1994, Kidney Int 45:224-231).

Many conditions resulting in a hypometabolic rate can produce a mild-to-moderate hypoproliferative anemia. Among such conditions are endocrine deficiency states. For example, anemia can occur in Addison's disease, hypothyroidism, hyperparathyroidism, or males who are castrated or treated with estrogen. Mild-to-moderate anemia can also occur with reduced dietary intake of protein, a condition particularly prevalent in the elderly. Finally, anemia can develop in patients with chronic liver disease arising from nearly any cause (Adamson, 2008, Harrison's Principles of Internal Medicine, 17th ed.; McGraw Hill, New York, pp 628-634).

Iron-deficiency anemia is the final stage in a graded progression of increasing iron deficiency which includes negative iron balance and iron-deficient erythropoiesis as intermediate stages. Iron deficiency can result from increased iron demand, decreased iron intake, or increased iron loss, as exemplified in conditions such as pregnancy, inadequate diet, intestinal malabsorption, acute or chronic inflammation, and acute or chronic blood loss. With mild-to-moderate anemia of this type, the bone marrow remains hypoproliferative, and RBC morphology is largely normal; however, even mild anemia can result in some microcytic hypochromic RBCs, and the transition to severe iron-deficient anemia is accompanied by hyperproliferation of the bone marrow and increasingly prevalent microcytic and hypochromic RBCs (Adamson, 2008, Harrison's Principles of Internal Medicine, 17th ed.; McGraw Hill, New York, pp 628-634). Appropriate therapy for iron-deficiency anemia depends on its cause and severity, with oral iron preparations, parenteral iron formulations, and RBC transfusion as major conventional options. An ActRIIb polypeptide, or other ActRIIb antagonist, could be used to treat chronic iron-deficiency anemias alone or in combination with conventional therapeutic approaches, particularly to treat anemias of multifactorial origin.

Hypoproliferative anemias can result from primary dysfunction or failure of the bone marrow, instead of dysfunction secondary to inflammation, infection, or cancer progression. Prominent examples would be myelosuppression caused by cancer chemotherapeutic drugs or cancer radiation therapy. A broad review of clinical trials found that mild anemia can occur in 100% of patients after chemotherapy, while more severe anemia can occur in up to 80% of such patients (Groopman et al., 1999, J Natl Cancer Inst 91:1616-1634). Myelosuppressive drugs include: 1) alkylating agents such as nitrogen mustards (e.g., melphalan) and nitrosoureas (e.g., streptozocin); 2) antimetabolites such as folic acid antagonists (e.g., methotrexate), purine analogs (e.g., thioguanine), and pyrimidine analogs (e.g., gemcitabine); 3) cytotoxic antibotics such as anthracyclines (e.g., doxorubicin); 4) kinase inhibitors (e.g., gefitinib); 5) mitotic inhibitors such as taxanes (e.g., paclitaxel) and vinca alkaloids (e.g., vinorelbine); 6) monoclonal antibodies (e.g., rituximab); and 7) topoisomerase inhibitors (e.g., topotecan and etoposide). An ActRIIb polypeptide, or other ActRIIb antagonist, can be used to treat anemia caused by chemotherapeutic agents and/or radiation therapy.

ActRIIb antagonists (e.g., ActRIIb polypeptides) would also be appropriate for treating anemias of disordered RBC maturation, which are characterized in part by undersized (microcytic), oversized (macrocytic), misshapen, or abnormally colored (hypochromic) RBCs.

Patients may be treated with a dosing regimen intended to restore the patient to a target hemoglobin level, usually between about 10 g/dl and about 12.5 g/dl, and typically about 11.0 g/dl (see also Jacobs et al. (2000) Nephrol Dial Transplant 15, 15-19), although lower target levels may cause fewer cardiovascular or other side effects. Alternatively, hematocrit levels (percentage of the volume of a blood sample occupied by the cells) can be used as a measure for the condition of red blood cells. Hematocrit levels for healthy individuals range from 41 to 51% for adult males and from 35 to 45% for adult females. Target hematocrit levels are usually around 30-33%. Moreover, hemoglobin/hematocrit levels vary from person to person. Thus, optimally, the target hemoglobin/hematocrit level can be individualized for each patient.

ActRIIb antagonists disclosed herein may be useful for increasing red blood cell and hemoglobin levels in patients that do not respond well to Epo. For example, an ActRIIb antagonist may be beneficial for a patient in which administering of a normal to increased (>300 IU/kg/week) dose of Epo does not result in the increase of hemoglobin level up to the target level. Patients with an inadequate Epo response are found for all types of anemia, but higher numbers of non-responders have been observed particularly frequently in patients with cancers and patients with end-stage renal disease. An inadequate response to Epo can be either constitutive (i.e. observed upon the first treatment with Epo) or acquired (e.g. observed upon repeated treatment with Epo).

The ActRIIb antagonists may also be used to treat patients that are susceptible to adverse effects of Epo. The primary adverse effects of Epo are an excessive increase in the hematocrit or hemoglobin levels and polycythemia. Elevated hematocrit levels can lead to hypertension (more particularly aggravation of hypertension) and vascular thrombosis. Other adverse effects of Epo which have been reported, some of which related to hypertension, are headaches, influenza-like syndrome, obstruction of shunts, myocardial infarctions and cerebral convulsions due to thrombosis, hypertensive encephalopathy, and red cell blood cell applasia (Singibarti, (1994) J. Clin Investig 72(suppl 6), S36-S43; Horl et al. (2000) Nephrol Dial Transplant 15(suppl 4), 51-56; Delanty et al. (1997) Neurology 49, 686-689; Bunn (2002) N Engl J Med 346(7), 522-523).

As described in U.S. Publication No. 2009/0005308, ActRIIb antagonists can be used to promote muscle growth and increase muscle strength. Thus, ActRIIb antagonists can be used to increase red blood cell levels and promote muscle growth. Thus, ActRIIb antagonists may be particularly helpful to patients with a disorder that is associated with muscle loss and anemia. Examples include cancer- and cancer treatments, many forms of cachexia (muscle wasting) and sarcopenia (muscle loss associated with aging).

### 7. Pharmaceutical Compositions

In certain embodiments, ActRIIb antagonists (e.g., ActRIIb polypeptides) of the present invention are formulated with a pharmaceutically acceptable carrier. For example, an ActRIIb polypeptide can be administered alone or as a component of a pharmaceutical formulation (therapeutic composition). The subject compounds may be formulated for administration in any convenient way for use in human or veterinary medicine.

In certain cases, the therapeutic method of the disclosure includes administering the composition systemically, or locally as an implant or device. When administered, the therapeutic composition for use in this disclosure is, of course, in a pyrogen-free, physiologically acceptable form. Therapeutically useful agents other than the ActRIIb antagonists which may also optionally be included in the composition as described above, may be administered simultaneously or sequentially with the subject compounds (e.g., ActRIIb polypeptides) in the methods of the disclosure.

Typically, ActRIIb antagonists will be administered parenterally. Pharmaceutical compositions suitable for parenteral administration may comprise one or more ActRIIb-polypeptides in combination with one or more pharmaceutically acceptable sterile isotonic aqueous or nonaqueous solutions, dispersions, suspensions or emulsions, or sterile powders which may be reconstituted into sterile injectable solutions or dispersions just prior to use, which may contain antioxidants, buffers, bacteriostats, solutes which render the formulation isotonic with the blood of the intended recipient or suspending or thickening agents. Examples of suitable aqueous and nonaqueous carriers which may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

Further, the composition may be encapsulated or injected in a form for delivery to a target tissue site (e.g., bone marrow). In certain embodiments, compositions of the present invention may include a matrix capable of delivering one or more therapeutic compounds (e.g., ActRIIb polypeptides) to a target tissue site (e.g., bone marrow), providing a structure for the developing tissue and optimally capable of being resorbed into the body. For example, the matrix may provide slow release of the ActRIIb polypeptides. Such matrices may be formed of materials presently in use for other implanted medical applications.

The choice of matrix material is based on biocompatibility, biodegradability, mechanical properties, cosmetic appearance and interface properties. The particular application of the subject compositions will define the appropriate formulation. Potential matrices for the compositions may be biodegradable and chemically defined calcium sulfate, tricalciumphosphate, hydroxyapatite, polylactic acid and polyanhydrides. Other potential materials are biodegradable and biologically well defined, such as bone or dermal collagen. Further matrices are comprised of pure proteins or extracellular matrix components. Other potential matrices are non-biodegradable and chemically defined, such as sintered hydroxyapatite, bioglass, aluminates, or other ceramics. Matrices may be comprised of combinations of any of the above mentioned types of material, such as polylactic acid and hydroxyapatite or collagen and tricalciumphosphate. The bioceramics may be altered in composition, such as in calcium-aluminate-phosphate and processing to alter pore size, particle size, particle shape, and biodegradability.

In certain cases, methods of the disclosure can be administered for orally, e.g., in the form of capsules, cachets, pills, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of an agent as an active ingredient. An agent may also be administered as a bolus, electuary or paste.

In solid dosage forms for oral administration (capsules, tablets, pills, dragees, powders, granules, and the like), one or more therapeutic compounds of the present invention may be mixed with one or more pharmaceutically acceptable carriers, such as sodium citrate or dicalcium phosphate, and/or any of the following: (1) fillers or extenders, such as starches, lactose, sucrose, glucose, mannitol, and/or silicic acid; (2) binders, such as, for example, carboxymethylcellulose, alginates, gelatin, polyvinyl pyrrolidone, sucrose, and/or acacia; (3) humectants, such as glycerol; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds; (7) wetting agents, such as, for example, cetyl alcohol and glycerol monostearate; (8) absorbents, such as kaolin and bentonite clay; (9) lubricants, such a talc, calcium stearate, magnesium stearate, solid polyethylene glycols, sodium lauryl sulfate, and mixtures thereof; and (10) coloring agents. In the case of capsules, tablets and pills, the pharmaceutical compositions may also comprise buffering agents. Solid compositions of a similar type may also be employed as fillers in soft and hard-filled gelatin capsules using such excipients as lactose or milk sugars, as well as high molecular weight polyethylene glycols and the like.

Liquid dosage forms for oral administration include pharmaceutically acceptable emulsions, microemulsions, solutions, suspensions, syrups, and elixirs. In addition to the active ingredient, the liquid dosage forms may contain inert diluents commonly used in the art, such as water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor, and sesame oils), glycerol, tetrahydrofuryl alcohol, polyethylene glycols and fatty acid esters of sorbitan, and mixtures thereof. Besides inert diluents, the oral compositions can also include adjuvants such as wetting agents, emulsifying and suspending agents, sweetening, flavoring, coloring, perfuming, and preservative agents.

Suspensions, in addition to the active compounds, may contain suspending agents such as ethoxylated isostearyl alcohols, polyoxyethylene sorbitol, and sorbitan esters, microcrystalline cellulose, aluminum metahydroxide, bentonite, agar-agar and tragacanth, and mixtures thereof.

The compositions of the invention may also contain adjuvants, such as preservatives, wetting agents, emulsifying agents and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption, such as aluminum monostearate and gelatin.

It is understood that the dosage regimen will be determined by the attending physician considering various factors which modify the action of the subject compounds of the invention (e.g., ActRIIb polypeptides). The various factors include, but are not limited to, the patient's red blood cell count, hemoglobin level or other diagnostic assessments, the desired target red blood cell count, the patient's age, sex, and diet, the severity of any disease that may be contributing to a depressed red blood cell level, time of administration, and other clinical factors. The addition of other known growth factors to the final composition may also affect the dosage. Progress can be monitored by periodic assessment of red blood cell and hemoglobin levels, as well as assessments of reticulocyte levels and other indicators of the hematopoietic process.

The present disclosure also provides gene therapy for the in vivo production of ActRIIb polypeptides. Such therapy would achieve its therapeutic effect by introduction of the ActRIIb polynucleotide sequences into cells or tissues having the disorders as listed above. Delivery of ActRIIb polynucleotide sequences can be achieved using a recombinant expression vector such as a chimeric virus or a colloidal dispersion system. Preferred for therapeutic delivery of ActRIIb polynucleotide sequences is the use of targeted liposomes.

Various viral vectors which can be utilized for gene therapy as taught herein include adenovirus, herpes virus, vaccinia, or an RNA virus such as a retrovirus. The retroviral vector may be a derivative of a murine or avian retrovirus. Examples of retroviral vectors in which a single foreign gene can be inserted include, but are not limited to: Moloney murine leukemia virus (MoMuLV), Harvey murine sarcoma virus (HaMuSV), murine mammary tumor virus (MuMTV), and Rous Sarcoma Virus (RSV). A number of additional retroviral vectors can incorporate multiple genes. All of these vectors can transfer or incorporate a gene for a selectable marker so that transduced cells can be identified and generated. Retroviral vectors can be made target-specific by attaching, for example, a sugar, a glycolipid, or a protein. Preferred targeting is accomplished by using an antibody. Those of skill in the art will recognize that specific polynucleotide sequences can be inserted into the retroviral genome or attached to a viral envelope to allow target specific delivery of the retroviral vector containing the ActRIIb polynucleotide.

Alternatively, tissue culture cells can be directly transfected with plasmids encoding the retroviral structural genes gag, pol and env, by conventional calcium phosphate transfection. These cells are then transfected with the vector plasmid containing the genes of interest. The resulting cells release the retroviral vector into the culture medium.

Another targeted delivery system for ActRIIb polynucleotides is a colloidal dispersion system. Colloidal dispersion systems include macromolecule complexes, nanocapsules, microspheres, beads, and lipid-based systems including oil-in-water emulsions, micelles, mixed micelles, and liposomes. The preferred colloidal system of this invention is a liposome. Liposomes are artificial membrane vesicles which are useful as delivery vehicles in vitro and in vivo. RNA, DNA and intact virions can be encapsulated within the aqueous interior and be delivered to cells in a biologically active form (see e.g., Fraley, et al., Trends Biochem. Sci., 6:77, 1981). Methods for efficient gene transfer using a liposome vehicle, are known in the art, see e.g., Mannino, et al., Biotechniques, 6:682, 1988. The composition of the liposome is usually a combination of phospholipids, usually in combination with steroids, especially cholesterol. Other phospholipids or other lipids may also be used. The physical characteristics of liposomes depend on pH, ionic strength, and the presence of divalent cations.

Examples of lipids useful in liposome production include phosphatidyl compounds, such as phosphatidylglycerol, phosphatidylcholine, phosphatidylserine, phosphatidylethanolamine, sphingolipids, cerebrosides, and gangliosides. Illustrative phospholipids include egg phosphatidylcholine, dipalmitoylphosphatidylcholine, and distearoylphosphatidylcholine. The targeting of liposomes is also possible based on, for example, organ-specificity, cell-specificity, and organelle-specificity and is known in the art.

### EXEMPLIFICATION

The invention now being generally described, it will be more readily understood by reference to the following examples, which are included merely for purposes of illustration of certain embodiments and embodiments of the present invention, and are not intended to limit the invention.

### Example 1. Generation of ActRIIb-Fc fusion proteins

Applicants constructed a soluble ActRIIb fusion protein that has the extracellular domain of human ActRIIb fused to a human or mouse Fc domain with a minimal linker (three glycine amino acids) in between. The constructs are referred to as ActRIIb-hFc and ActRIIb-mFc, respectively.

ActRIIb-hFc is shown below as purified from CHO cell lines (SEQ ID NO: 8):

GRGEAETRECIYYNANWELERTNQSGLERCEGEQDKRLHCYASWRNSSGTI
ELVKKGCWLDDFNCYDRQECVATEENPQVYFCCCEGNFCNERFTHLPEAG
GPEVTYEPPPTAPTGGGTHTCPPCPAPELLGGPSVFLFPPKPKDTLMISRTPEV
TCVVVDVSHEDPEVKFNWYVDGVEVHNAKTKPREEQYNSTYRVVSVLTVL
HQDWLNGKEYKCKVSNKALPVPIEKTISKAKGQPREPQVYTLPPSREEMTK
NQVSLTCLVKGFYPSDIAVEWESNGQPENNYKTTPPVLDSDGSFFLYSKLTV
DKSRWQQGNVFSCSVMHEALHNHYTQKSLSLSPGK

The ActRIIb-hFc and ActRIIb-mFc proteins were expressed in CHO cell lines. Three different leader sequences were considered:
(i) Honey bee mellitin (HBML): MKFLVNVALVFMVVYISYIYA (SEQ ID NO: 11)
(ii) Tissue Plasminogen Activator (TPA): MDAMKRGLCCVLLLCGAVFVSP (SEQ ID NO: 12)
(iii) Native: MGAAAKLAFAVFLISCSSGA (SEQ ID NO: 13).

The selected form employs the TPA leader and has the following unprocessed amino acid sequence (SEQ ID NO: 9):

MDAMKRGLCCVLLLCGAVFVSPGASGRGEAETRECIYYNANWELERTNQS
GLERCEGEQDKRLHCYASWRNSSGTIELVKKGCWLDDFNCYDRQECVATE
ENPQVYFCCCEGNFCNERFTHLPEAGGPEVTYEPPPTAPTGGGTHTCPPCPA
PELLGGPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPVPIE
KTISKAKGQPREPQVYTLPPSREEMTKNQVSLTCLVKGFYPSDIAVEWESNG
QPENNYKTTPPVLDSDGSFFLYSKLTVDKSRWQQGNVFSCSVMHEALHNH
YTQKSLSLSPGK

This polypeptide is encoded by the following nucleic acid sequence (SEQ ID NO:10):

A TGGATGCAAT GAAGAGAGGG CTCTGCTGTG TGCTGCTGCT GTGTGGAGCA
GTCTTCGTTT CGCCCGGCGC CTCTGGGCGT GGGGAGGCTG AGACACGGGA
GTGCATCTAC TACAACGCCA ACTGGGAGCT GGAGCGCACC AACCAGAGCG

GCCTGGAGCG CTGCGAAGGC GAGCAGGACA AGCGGCTGCA CTGCTACGCC
TCCTGGCGCA ACAGCTCTGG CACCATCGAG CTCGTGAAGA AGGGCTGCTG
GCTAGATGAC TTCAACTGCT ACGATAGGCA GGAGTGTGTG GCCACTGAGG
AGAACCCCCA GGTGTACTTC TGCTGCTGTG AAGGCAACTT CTGCAACGAG
CGCTTCACTC ATTTGCCAGA GGCTGGGGGC CCGGAAGTCA CGTACGAGCC
ACCCCCGACA GCCCCCACCG GTGGTGGAAC TCACACATGC CCACCGTGCC
CAGCACCTGA ACTCCTGGGG GGACCGTCAG TCTTCCTCTT CCCCCCAAAA
CCCAAGGACA CCCTCATGAT CTCCCGGACC CCTGAGGTCA CATGCGTGGT
GGTGGACGTG AGCCACGAAG ACCCTGAGGT CAAGTTCAAC TGGTACGTGG
ACGGCGTGGA GGTGCATAAT GCCAAGACAA AGCCGCGGGA GGAGCAGTAC
AACAGCACGT ACCGTGTGGT CAGCGTCCTC ACCGTCCTGC ACCAGGACTG
GCTGAATGGC AAGGAGTACA AGTGCAAGGT CTCCAACAAA GCCCTCCCAG
TCCCCATCGA GAAAACCATC TCCAAAGCCA AAGGGCAGCC CCGAGAACCA
CAGGTGTACA CCCTGCCCCC ATCCCGGGAG GAGATGACCA AGAACCAGGT
CAGCCTGACC TGCCTGGTCA AAGGCTTCTA TCCCAGCGAC ATCGCCGTGG
AGTGGGAGAG CAATGGGCAG CCGGAGAACA ACTACAAGAC CACGCCTCCC
GTGCTGGACT CCGACGGCTC CTTCTTCCTC TATAGCAAGC TCACCGTGGA
CAAGAGCAGG TGGCAGCAGG GGAACGTCTT CTCATGCTCC GTGATGCATG
AGGCTCTGCA CAACCACTAC ACGCAGAAGA GCCTCTCCCT GTCTCCGGGT
AAATGA

N-terminal sequencing of the CHO-cell produced material revealed a major sequence of -GRGEAE (SEQ ID NO: 22). Notably, other constructs reported in the literature begin with an -SGR... sequence.

Purification could be achieved by a series of column chromatography steps, including, for example, three or more of the following, in any order: protein A chromatography, Q sepharose chromatography, phenylsepharose chromatography, size exclusion chromatography, and cation exchange chromatography. The purification could be completed with viral filtration and buffer exchange.

ActRIIb-Fc fusion proteins were also expressed in HEK293 cells and COS cells. Although material from all cell lines and reasonable culture conditions provided protein with muscle-building activity in vivo, variability in potency was observed perhaps relating to cell line selection and/or culture conditions.

Applicants generated a series of mutations in the extracellular domain of ActRIIB and produced these mutant proteins as soluble fusion proteins between extracellular ActRIIB and an Fc domain. The background ActRIIB-Fc fusion has the sequence of SEQ ID NO:6. Various mutations, including N- and C-terminal truncations, were introduced into the background ActRIIB-Fc protein. Based on the data presented in Example 1, it is expected that these constructs, if expressed with a TPA leader, will lack the N-terminal serine. Mutations were generated in ActRIIB extracellular domain by PCR mutagenesis. After PCR, fragments were purified through a Qiagen column, digested with SfoI and AgeI and gel purified. These fragments were ligated into expression vector pAID4 (see WO2006/012627) such that upon ligation it created fusion chimera with human IgG1. Upon transformation into E. coli DH5 alpha, colonies were picked and DNAs were isolated. For murine constructs (mFc), a murine IgG2a was substituted for the human IgG1. All mutants were sequence verified.

All of the mutants were produced in HEK293T cells by transient transfection. In summary, in a 500ml spinner, HEK293T cells were set up at 6x10⁵ cells/ml in Freestyle (Invitrogen) media in 250ml volume and grown overnight. Next day, these cells were treated with DNA:PEI (1:1) complex at 0.5 ug/ml final DNA concentration. After 4 hrs, 250 ml media was added and cells were grown for 7 days. Conditioned media was harvested by spinning down the cells and concentrated.

Mutants were purified using a variety of techniques, including, for example, protein A column and eluted with low pH (3.0) glycine buffer . After neutralization, these were dialyzed against PBS.

Mutants were also produced in CHO cells by similar methodology.

Mutants were tested in binding assays and/or bioassays described. Characteristics of various ActRIIb variants are described in WO/2008/097541 and WO/2006/012627. In some instances, assays were performed with conditioned medium rather than purified proteins. Additional variations of ActRIIb are described in US Application Serial No. 12/012,652.

### Example 2. ActRIIb-hFc Stimulates Erythropoiesis in Non-Human Primates

ActRIIb-hFc (IgG1) was administered once a week for 1-month to male and female cynomolgus monkeys by subcutaneous injection. Forty-eight cynomolgus monkeys (24/sex) were assigned to one of four treatment groups (6 animals/sex/group) and were administered subcutaneous injections of either vehicle or ActRIIb-hFc at 3, 10, or 30 mg/kg once weekly for 4 weeks (total of 5 doses). Parameters evaluated included general clinical pathology (hematology, clinical chemistry, coagulation, and urinalysis). ActRIIb-hFc caused statistically significant elevated mean absolute reticulocyte values by day 15 in treated animals. By day 36, ActRIIb-hFc caused several hematological changes, including elevated mean absolute reticulocyte and red blood cell distribution width values and lower mean corpuscular hemoglobin concentration. All treated groups and both sexes were affected. These effects are consistent with a positive effect of ActRIIb-hFc on the release of immature reticulocytes from the bone marrow. This effect was reversed after drug was washed out of the treated animals (by study day 56). Accordingly, we conclude that ActRIIb-hFc stimulates erythropoiesis.

### Example 3. ActRIIb-mFc Promotes Aspects of Erythropoiesis in Mice by Stimulation of Splenic Erythropoietic Activities

In this study the effects of the in vivo administration of ActRIIb-mFc on the frequency of hematopoietic progenitors in bone marrow and spleen was analyzed. One group of Black6 mice was injected with PBS as a control and a second group of mice administered two doses of ActRIIb-mFc at 10 mg/kg and both groups sacrificed after 8 days. Peripheral blood was used to perform complete blood counts and femurs and spleens were used to perform in vitro clonogenic assays to assess the lymphoid, erythroid and myeloid progenitor cell content in each organ. In the brief time frame of this study, no significant changes were seen in red blood cell, hemoglobin or white blood cell levels in treated mice. In the femurs there was no difference in the nucleated cell numbers or progenitor content between the control and treated groups. In the spleens, the compound treated group experienced a statistically significant increase in the mature erythroid progenitor (CFU-E) colony number per dish, frequency and total progenitor number per spleen. In addition, and increase was seen in the number of myeloid (CFU-GM), immature erythroid (BFU-E) and total progenitor number per spleen.

Except for the strain of mouse used, the detailed methodology in this study was the same as that described above in Example 6. Mean values (+/- SD) for each group are shown in the tables below.

**Table: Hematologic Parameters**

| Treatment Group | White Blood Cells (x10⁹/L) | Red Blood Cells (x10⁹/L) | Hemoglobin (g/L) | Hematocrit (L/L) |
|---|---|---|---|---|
| PBS (n=8) | 9.53 +/- 1.44 | 10.5 +/- 1.1 | 160.9 +/- 13.3 | 0.552 +/- 0.057 |
| ActRIIb-mFc (n=8) | 9.77 +/- 1.19 | 10.8 +/- 0.3 | 162.1 +/- 4.1 | 0.567 +/- 0.019 |

**Table: CFC From Femur and Spleen**

| Treatment Group | Total CFC per Femur | Total CFC per Spleen | Total CFU-E per Femur | Total CFU-E per Spleen |
|---|---|---|---|---|
| PBS (n=8) | 88 +/- 10 | 54 +/- 14 | 156 +/- 27 | 131 +/- 71 |
| ActRIIb-mFc (n=8) | 85 +/- 9 | 79 +/- 6* | 164 +/- 23 | 436 +/- 86* |

| | | | | |
|---|---|---|---|---|
| * preliminary analysis indicates p<0.05 | | | | |

Treatment of mice with ActRIIb-mFc, in the brief time frame of this study, did not result in significant increases in red blood cell or hemoglobin content. In the femurs there was no difference in the nucleated cell numbers or progenitor content between the control and treated groups. In the spleens, the compound treated group experienced a statistically significant increase in the nucleated cell number before red blood cell lysis and in the mature erythroid progenitor (CFU-E) colony number per dish, frequency and total progenitor number per spleen. In addition, an increase was seen in the number of myeloid (CFU-GM), immature erythroid (BFU-E) and total progenitor number per spleen. Accordingly, it is expected that over a longer time course, ActRIIb-mFc treatment may result in elevated red blood cell and hemoglobin content.

### Example 4: Effects of ActRIIb-Fc on Various Species in Longer-Term Studies

ActRIIb-Fc has a statistically significant effect on hematologic parameters in rodents. In a 3-month multidose study of ActRIIb-hFc in rats, significant increases in hemoglobin concentration or RBC count were observed, and reticulocyte concentrations increased in a dose-dependent manner.

**Table: Hematologic parameters in 3-month study in Sprague-Dawley rats**

| Sex (n) | **Males** (10/group) | | | |
|---|---|---|---|---|
| Dose (mg/kg) | **Vehicle** | **3** | **10** | **60** |
| RBC (x 10⁶/µL) | 8.6 | **9.9 *** | **10.2 *** | **9.1 *** |
| Hemoglobin (g/dL) | 15.9 | **17.4 *** | **17.9 *** | 16.4 |
| Reticulocytes (x 10⁹/L) | 176 | **250 *** | **272 *** | **446 *** |
| | | | | |

| Sex (n) | **Females** (10/group) | | | |
|---|---|---|---|---|
| Dose (mg/kg) | **Vehicle** | **3** | **10** | **30** |
| RBC (x 10⁶/µL) | 8.2 | 8.7 | **9.3** * | **9.7** * |
| Hemoglobin (g/dL) | 15.7 | 16.2 | 16.5 | 17.5 |
| Reticulocytes (x 10⁹/L) | 169 | 200 | 239 | 332 * |

| | | | | |
|---|---|---|---|---|
| * Statistically significant vs. vehicle (P ≤ 0.05) | | | | |

Interestingly, in a 3-month multidose study of ActRIIB-hFc in cynomolgus monkeys there were no significant increases in hematocrit levels, hemoglobin levels, or RBC count, and reticulocyte concentrations increased modestly over the course of the study. In a Phase Ia trial of ActRIIB-hFC, there were increases in hematologic parameters at some doses, with elevations typically observed at the highest dose levels within days of the first dose and at study completion. These data indicate that ActRIIB-Fc fusion proteins can be used to increase hematologic parameters in humans.

### Example 5: ActRIIb-mFc Increases Muscle Mass in Mice

As described in U.S. Pat. Appl. 12/012,652, ActRIIb-mFc is effective to promote growth of muscle mass in a variety of mouse models of human muscle disorders, including muscle dystrophy, amyotrophic lateral sclerosis and cancer cachexia. As an example, one set of this data is presented here.
Applicants tested the ability of ActRIIB (R64 20-134)-mFc to attenuate muscle loss in a mouse model of glucocorticoid-induced muscle wasting.

Mice were subcutaneously dosed daily for 13 days with either PBS or dexamethasone (2mg/kg) to induce muscle wasting. Over the same 13 days, PBS- and dexamethosone-treated groups received vehicle or ActRIIB (R64 20-134)-mFc (10mg/kg; i.p.; twice/week) such that all combinations of treatments were represented. Mice were NMR scanned at days 0 and 13 to determine changes in lean tissue mass across the groups. NMR results are outlined in Table 6, below.

**Table 6: Lean tissue mass of vehicle- and murine ActRIIB (R64 20-134)-Fc -treated mice**

| Group (sc:ip treatment) | | Avg lean day 13-Avg lean day 0 (g) ± std dev |
|---|---|---|
| PBS:PBS | | 0.83 ±0.94 |
| Dexameth:PBS | | 0.47 ±0.34^{a} |
| Dexameth:ActRIIB | | 2.56 ±0.37^{a,b} |
| PBS :ActRIIB | | 3.63 ±0.62^{a} |

| | | |
|---|---|---|
| ^{a} Significant difference compared to PBS:PBS at p < 0.05 ^{b} Significant difference compared to Dexameth:PBS at p < 0.05 | | |

NMR scanning showed a significant 2.5% decrease in lean tissue mass in the dexamethasone:PBS group compared to the PBS:PBS cohort. In contrast, the dexamethasone: ActRIIB (R64 20-134)-mFc group exhibited a 13.5% increase in lean tissue mass, a significant increase when compared to both the PBS:PBS and the dexamethasone:PBS groups. Cachexia is an undesirable side effect for a variety of therapeutic treatments, including chronic glucocorticoid therapy. Therefore it could be of clinical importance that treatment with a human ActRIIB (R64 20-134)-mFc protein can attenuate the muscle wasting associated with cachexia.

While specific embodiments of the subject matter have been discussed, the above specification is illustrative and not restrictive. Many variations will become apparent to those skilled in the art upon review of this specification and the claims below. The full scope of the invention should be determined by reference to the claims, along with their full scope of equivalents, and the specification, along with such variations.

## Claims

1. A composition for use in increasing red blood cell levels and increasing muscle formation in an anemia patient in need thereof, wherein (i) the patient has cachexia or sarcopenia, (ii) the composition comprises an effective amount of an ActRIIb antagonist and (iii) the antagonist is a fusion protein comprising an immunogloblin Fc domain and an ActRIIb polypeptide, the ActRIIb polypeptide being selected from the group consisting of:
a) a polypeptide comprising an amino acid sequence at least 90% identical to SEQ ID NO: 1;
b) a polypeptide comprising an amino acid sequence at least 90% identical to SEQ ID NO: 2; and
c) a polypeptide comprising an amino acid sequence at least 90% identical to SEQ ID NO: 3

2. The composition for use of claim 1, wherein the ActRIIb antagonist comprises an amino acid sequence at least 95% identical to SEQ ID NO: 2, SEQ ID NO: 3, SEQ ID NO: 6; SEQ ID NO: 8 or SEQ ID NO: 9.

3. The composition for use of claim 2, wherein the ActRIIb antagonist comprises the amino acid sequence of SEQ ID NO: 2, SEQ ID NO: 3, SEQ ID NO: 6; SEQ ID NO: 8 or SEQ ID NO: 9.

4. The composition for use of any preceding claim, wherein said ActRIIb antagonist includes one or more modified amino acid residues selected from: a glycosylated amino acid, a PEGylated amino acid, a farnesylated amino acid, an acetylated amino acid, a biotinylated amino acid, an amino acid conjugated to a lipid moiety, and an amino acid conjugated to an organic derivatizing agent.

5. The composition for use of claim 4, wherein the ActRIIb-Fc antagonist comprises the amino acid sequence of SEQ ID NO: 8 or SEQ ID NO: 9.

6. The composition for use of any preceding claim, wherein the patient has cachexia.

7. The composition for use of any one of claims 1-5, wherein the patient has sarcopenia.

## Patentansprüche

1. Zusammensetzung zur Verwendung bei der Erhöhung der Konzentration an roten Blutkörperchen und der Steigerung der Muskelbildung bei einem anämischen Patienten der dessen bedarf, wobei (i) der Patient Kachexie oder Sarkopenie aufweist, (ii) die Zusammensetzung eine wirksame Menge eines ActRIIb-Antagonisten umfasst und (iii) der Antagonist ein Fusionsprotein ist, das eine Immunglobulin-Fc-Domäne und ein ActRIIb-Polypeptid umfasst, wobei das ActRIIb-Polypeptid aus der Gruppe ausgewählt ist, die aus Folgendem besteht:
(a) einem Polypeptid, umfassend eine Aminosäuresequenz, die wenigstens 90 % identisch mit SEQ ID NO: 1 ist;
(b) einem Polypeptid, umfassend eine Aminosäuresequenz, die wenigstens 90 % identisch mit SEQ ID NO: 2 ist; und
(c) einem Polypeptid, umfassend eine Aminosäuresequenz, die wenigstens 90 % identisch mit SEQ ID NO: 3 ist.

2. Zusammensetzung zur Verwendung nach Anspruch 1, wobei der ActRIIb-Antagonist eine Aminosäuresequenz umfasst, die wenigstens 95 % identisch mit SEQ ID NO: 2, SEQ ID NO: 3, SEQ ID NO: 6, SEQ ID NO: 8, oder SEQ ID NO: 9 ist.

3. Zusammensetzung zur Verwendung nach Anspruch 2, wobei der ActRIIb-Antagonist die Aminosäuresequenz der SEQ ID NO: 2, SEQ ID NO: 3, SEQ ID NO: 6, SEQ ID NO: 8 oder SEQ ID NO: 9 umfasst.

4. Zusammensetzung nach einem der vorhergehenden Ansprüche, wobei der ActRIIb-Antagonist einen oder mehrere modifizierte Aminosäurereste umfasst, ausgewählt aus: einer glykosylierten Aminosäure, einer PEGylierten Aminosäure, einer farnesylierten Aminosäure, einer acetylierten Aminosäure, einer biotinylierten Aminosäure, einer mit einem Lipidteil konjugierten Aminosäure, und einer Aminosäure, die mit einem organischen Derivatisierungsmittel konjugiert ist.

5. Zusammensetzung zur Verwendung nach Anspruch 4, wobei der ActRIIb-Fc-Antagonist die Aminosäuresequenz der SEQ ID NO: 8 oder SEQ ID NO: 9 umfasst.

6. Zusammensetzung zur Verwendung nach einem der vorhergehenden Ansprüche, wobei der Patient Kachexie aufweist.

7. Zusammensetzung zur Verwendung nach einem der Ansprüche 1-5, wobei der Patient Sarkopenie aufweist.

## Revendications

1. Composition pour utilisation dans l'augmentation des taux de globules rouges et l'augmentation de la formation de muscle chez un patient anémique en ayant besoin, où (i) le patient est atteint de cachexie ou de sarcopénie, (ii) la composition comprend une quantité efficace d'un antagoniste d'ActRIIb et (iii) l'antagoniste est une protéine de fusion comprenant un domaine Fc d'immunoglobuline et un polypeptide ActRIIb, le polypeptide ActRIIb étant choisi dans le groupe constitué de :
a) un polypeptide comprenant une séquence d'acides aminés au moins 90 % identique à SEQ ID NO : 1 ;
b) un polypeptide comprenant une séquence d'acides aminés au moins 90 % identique à SEQ ID NO : 2 ; et
c) un polypeptide comprenant une séquence d'acides aminés au moins 90 % identique à SEQ ID NO : 3.

2. Composition pour utilisation selon la revendication 1, dans laquelle l'antagoniste d'ActRIIb comprend une séquence d'acides aminés au moins 95 % identique à SEQ ID NO : 2, SEQ ID NO : 3, SEQ ID NO : 6 ; SEQ ID NO : 8 ou SEQ ID NO : 9.

3. Composition pour utilisation selon la revendication 2, dans laquelle l'antagoniste d'ActRIIb comprend la séquence d'acides aminés de SEQ ID NO : 2, SEQ ID NO : 3, SEQ ID NO : 6 ; SEQ ID NO : 8 ou SEQ ID NO : 9.

4. Composition pour utilisation selon l'une quelconque des revendications précédentes, dans laquelle ledit antagoniste d'ActRIIb comprend un ou plusieurs résidus d'acide aminé modifiés choisis parmi : un acide aminé glycosylé, un acide aminé PEGylé, un acide aminé farnésylé, un acide aminé acétylé, un acide aminé biotinylé, un acide aminé conjugué à un fragment lipidique, et un acide aminé conjugué à un agent de dérivation organique.

5. Composition pour utilisation selon la revendication 4, dans laquelle l'antagoniste d'ActRIIb-Fc comprend la séquence d'acides aminés de SEQ ID NO : 8 ou SEQ ID NO : 9.

6. Composition pour utilisation selon l'une quelconque des revendications précédentes, le patient étant atteint de cachexie.

7. Composition pour utilisation selon l'une quelconque des revendications 1 à 5, le patient étant atteint de sarcopénie.
